# EUROPÄISCHE PATENTSCHRIFT

(11) **EP 4 008 009 B1**
(45) Veröffentlichungstag und Bekanntmachung des Hinweises auf die Patenterteilung: **18.12.2024**
(21) Anmeldenummer: 20749891.6
(22) Anmeldetag: 30.07.2020
(51) Int. Cl.: G16H 20/17, G16H 40/63, A61M 39/10

(54) **INTELLIGENTES MEDIZINISCHES FLUIDLEIT- UND -TRANSFERSYSTEM SOWIE KOMPONENTEN HIERFÜR**
INTELLIGENT MEDICAL FLUID CONDUCTION AND TRANSFER SYSTEM, AND COMPONENTS THEREFOR
SYSTÈME INTELLIGENT DE GUIDAGE ET DE TRANSFERT DE FLUIDE MÉDICAL, ET ÉLÉMENTS ASSOCIÉS

(30) Priorität: 02.08.2019 DE 102019120926
(43) Veröffentlichungstag der Anmeldung: 08.06.2022
(73) Patentinhaber: ConnCons GmbH, 01445 Radebeul (DE)
(72) Erfinder: SCHURIG, Carsten, 01445 Radebeul (DE); HAMPE, Jochen, 01277 Dresden (DE); GOMMEL, Christoph, 01445 Radebeul (DE)
(74) Vertreter: Witte, Weller und Partner Patentanwälte mbB Stuttgart
(86) Internationale Anmeldenummer: PCT/EP2020/071596
(87) Internationale Veröffentlichungsnummer: WO 2021/023639

(56) Entgegenhaltungen:
- DE-A1- 102011 088 505
- US-A1- 2009 327 715
- US-A1- 2015 265 828
- US-A1- 2015 306 365
- US-A1- 2016 073 929
- US-A1- 2016 114 104
- US-A1- 2018 161 568
- US-A1- 2018 236 204

## Beschreibung

### ANWENDUNGSGEBIET UND STAND DER TECHNIK

Die Erfindung betrifft ein medizinisches Fluidleit- und -transfersystem sowie Komponenten, die in einem solchen System Verwendung finden können.

Ein medizinisches Fluidleit- und -transfersystem ist ein System zur Führung von Fluiden im Zusammenhang mit der medizinischen Behandlung eines Menschen oder eines Tieres. Ein solches System kann beispielsweise ein Infusionssystem oder ein Transfusionssystem oder ein Dialysesystem oder ein System zum extrakorporalen Gasaustauch oderein extrakorporales Kreislaufunterstützungssystem oder eine Herz-Lungen-Maschine oder ein Ernährungssystem oder ein Drainagesystem für Körperflüssigkeiten, wie Urin, Wundsekret oder Darminhalt oder ein Beatmungssystem sein.

Ein gattungsgemäßes ebenso wie ein erfindungsgemäßes Fluidleit- und -transfersystem verfügt üblicherweise über mindestens eine Fluidquelle zur Bereitstellung eines medizinisch relevanten Fluidesoder Fluidgemisches, übereine Zugangseinheit zum Applizieren des medizinisch relevanten Fluids oder Fluidgemisches in einem Patienten sowie über ein Verbindungsleitersystem, welches die Fluidquelle und die Zugangseinheit verbindet und zum Leiten und Transferieren des medizinisch relevanten Fluidgemisches von der Fluidquelle zum Patienten ausgebildet ist, wobei das Verbindungsleitersystem meist mindestens ein regulierendes Transferelement aufweist.

Ein Fluid oder Fluidgemisch im Sinne der Erfindung kann gasförmig sein, z. B. Atemluft, Umgebungsluft angereichert mit Narkosegas, Sauerstoff; oder es kann flüssig sein, z. B. eine Infusionslösung, ein Medikament, Urin, Blut, ein Dialysat oder Vorstufen der Dialysate.

Als Fluidquelle wird eine Speichereinheit oder eine Medienversorgung für das Fluid oder Fluidgemisch, beispielsweise Wandanschlüsse für Sauerstoff oder natürliche Quellen, verstanden.

Eine Zugangseinheit kann beispielsweise durch eine Nadel oder einen temporären Gefäßzugang - wie einen zentralen Venenkatheter oder einen arteriellen Zugang gebildet werden, die bzw. der in eine Vene oder Arterie des Patienten eingeführt wird. Eine Zugangseinheit kann auch durch einen Portgebildetsein, d. h. ein subkutan implantiertes Kathetersystem, das von außen punktiert werden kann und einen dauerhaften Zugangzum arteriellen oder venösen Gefäßsystem oderzu bestimmten Körperhöhlen bietet.

Das Verbindungsleitersystem im Sinne der Erfindung fasst alle Komponenten des Fluidleit- und - transfersystems zusammen, die zum Leiten und Transferieren bzw. Verteilen des medizinisch relevanten Fluids oder Fluidgemisches notwendig sind. Ein regulierendes Transferelement wie beispielsweise ein Dreiwegehahn, eine Hahnbank oder ein Filterelement ist Teil des Verbindungsleitersystems im Sinne der Erfindung. Unter einem Transferelement wird dabei ein Element verstanden, welches dazu dient, medizinisch relevante Fluide oder Fluidgemische aus mehreren mit dem Transferelement über jeweils einen Verbindungsleiter des Verbindungsleitersystems verbundenen Fluidquellen gleichzeitig in eine Zugangseinheit am Patienten weiterzuleiten bzw. zu transferieren.

Praktisch jeder stationäre Patient erhält über eine Zugangseinheit - d. h. intravenöse (IV) periphere Verweilkanülen, zentrale temporäre Verweilkatheter oder permanent implantierte Gefäßzugänge (sogenannte "Ports") - Medikamente, Chemotherapie, Infusionsflüssigkeiten oder Ernährungslösungen verabreicht. Man spricht dabei auch von IV-Therapien. Fast alle diese Therapien werden bereits heute schon über meist offline programmierbare Pumpen - z. B. Infusionspumpen in Infusionssystemen - durchgeführt, d. h. die Pumpenparameter werden entsprechend ihrer Verwendung am Patienten voreingestellt und mit dem Patienten verbunden.

Bei Patienten auf Intensivstationen, in Narkose oder mit schwerer Erkrankung wird oft eine Vielzahl von IV-Therapien gleichzeitig über verschiedene IV-Zugänge durchgeführt. Die Anzahl kann bis zu 30 betragen. In diesen Situationen sind mehrere parallele intravenöse Zugänge notwendig, um präzise steuerbar lebenserhaltende und untereinander inkompatible Medikamente kontrolliert zu applizieren. Die korrekte Verabreichung und Dokumentation dieser Therapien ist hoch relevant für die Patientensicherheit und die medikolegale Situation der behandelnden Pflegekräfte, Ärzte und Einrichtungen. Zur Durchführung dieser Therapien werden jährlich Milliarden von Einwegartikeln hergestellt.

Die heutigen Infusions- und Verbindungssysteme sind Ende des 19. Jahrhunderts erdacht worden und sind insbesondere auf eine gute Funktionsfähigkeit bei reinem Schwerkraftbetrieb ausgerichtet. Die Fortbewegung der Infusionsflüssigkeit kann aber auch mittels einer Infusionspumpe oder durch aktiven Flüssigkeitsausstrom aus der Fluidquelle - beispielsweise aus einem Vorratsbehälter für die Infusionsflüssigkeit - realisiert werden. Auf dem Weg zur Zugangseinheit, z. B. einem IV-Zugang am Patienten, können ein oder mehrere regulierende Transferelemente ausgebildet sein. Das für IV-Therapien international nahezu ausschließlich genutzte Luer-System geht auf den Instrumentenmacher Hermann Wülfing Luer (11883) zurück. Die Kombination der Luer-Verbindung mit einer Drehsicherung (Luer-Lock, als Weiterentwicklung der Steckverbindung Luer-Slip) wurde erstmals 1952 beschrieben. Das System ist in der ISO-Norm ISO 80369-7 international standardisiert. In seiner jetzigen Form werden die Komponenten des Infusionssystems bzw. eines Fluidleit- und -transfersystems manuell zusammengesteckt oder geschraubt und manuell auf ihre physisch korrekte Verbindung kontrolliert.

Überein Verbindungsleitersystem, welches bei einem Infusionssystem durch die Infusionsleitungen oder -schläuche gebildet wird, die Infusionspumpe und die regulierenden Transferelemente erfolgt der Transport der Infusionsflüssigkeiten von einem Vorratsbehälter zu einem IV-Zugang am Patienten.

Für ein funktionsfähiges und medizinisch wirksames Fluidleit- und -transfersystem ist es essenziell, dass alle Komponenten korrekt funktionieren und miteinander verbunden sind. Bei einem Infusionssystem muss beispielsweise gewährleistet sein, dass das richtige Medikament über eine entsprechende Infusionspumpe mit der richtigen Infusionsrate verabreicht wird und es muss eine durchgängige und Kompatibilitäts- und Sicherheitskriterien entsprechende Verbindung bis in den Patienten sichergestellt sein, so dass beispielsweise inkompatible Medikamente und Infusionslösungen nicht gemeinsam verabreicht werden können. Mit den bisher bekannten Infusionssystemen erfolgt die Kontrolle der korrekten Verbindung manuell durch den Arzt oder eine Pflegekraft.

Für die sichere Funktion eines medizinischen Fluidleitsystems ist die Beachtung der Hygienestandards zum Schutz des Patienten essenziell. Dies betrifft insbesondere die korrekte Verwendung von Filtern, das Vermeiden unnötiger Rekonnektierungen und Einhaltung der Wechselintervalle. Bisher bekannte Fluidleitsysteme erlauben keine Prüfung oder Überwachung dieser Parameter. Stattdessen erfolgt die Kontrolle und Dokumentation manuell durch den Arzt oder eine Pflegekraft.

Von medizinischen Fluidleitsystemen kann eine erhebliche Gefahr für Mitpatienten, Angehörige oder Pflegekraft ausgehen. Dies ist beispielsweise der Fall, wenn das Fluidleitsystem mit hoch toxischen Stoffen, wie bei der Chemotherapie, gefüllt ist und durch Leckage oder Bedienfehler diese Stoffe austreten. Der Austritt von Chemotherapeutikum kann von bisherigen Systemen nicht erkannt werden.

Des Weiteren ist mit den bisher verwendeten Fluidleit- und -transfersystemen eine Therapiedokumentation, Integritätsprüfung, Plausibilitätstestung oder Rückmeldung über den Status und Probleme im Rahmen z. B. der Infusionstherapie nicht möglich. Unter diesen Einschränkungen leidet die Patientensicherheit, es entsteht ein hoher manueller Dokumentationsaufwand. Eine elektronische Verzahnung z. B. der Infusionstherapie mit den übrigen Therapieabläufen ist nicht möglich.

Im Stand der Technik sind bereits verschiedene Vorschläge zur Integration von Datenleitungen mit Fluidleitungen gemacht worden.

Das Dokument US 2009/0327715 A1 behandelt die kryptografische Identifizierung austauschbarer Teile, insbesondere in medizinischen Geräten wie Schlauchsystemen. Es beschreibt Fluidleitungen mit integrierten Datenleitungen, bei denen eine elektrische Verbindung über eine Ein-Draht-Kommunikationsleitung und Erdung nur dann hergestellt wird, wenn eine mechanische Verbindung zwischen den Fluidleitungen besteht.

Das Dokument US 2015/0265828 A1 stellt Adapter vor, die in medizinischen Schlauchverbindungen verwendet werden, um eine fehlerfreie Verbindung zwischen medizinischen Geräten und Schläuchen sicherzustellen. Diese Adapter gewährleisten, dass ein medizinisches Gerät nur aktiviert wird, wenn die Schlauchverbindung korrekt hergestellt ist. Dies wird durch integrierte Leitungen und optische Systeme erreicht, die den Verbindungsstatus erkennen und die Datenübertragung ermöglichen.

Das Dokument US 2018/0161568 A1 beschreibt ein Fluidanschlusssystem für medizinische Anwendungen, das die sichere Verbindung von Fluidleitungen und den Schutz vor Verunreinigungen gewährleistet. Es beinhaltet einen Mechanismus mit mechanischen oder elektronischen Schlüsseln, die eine Sicherheitsüberprüfung vor der Aktivierung der Verbindung ermöglichen.

Das Dokument US 2015/0306365 A1 beschreibt Fluid-Steckverbinder, die elektrische Elemente wie Widerstände und Kondensatoren enthalten, die zwischen zwei leitenden Elementen angeordnet sind. Diese Schaltung wird geschlossen, wenn der Steckverbinder mit einem medizinischen Gerät verbunden wird. Die elektrischen Komponenten dienen dazu, die Verbindung zu identifizieren und das Gerät zu aktivieren oder den Betriebsmodus anzupassen.

Das Dokument US 2018/0236204 A1 befasst sich mit einem Katheteranschluss, der eine Reihe von Kontaktkomponenten enthält, die mit einem passenden Kabel verbunden werden können, das über eine entsprechende zweite Reihe von Kontaktkomponenten verfügt.

Das Dokument DE 10 2011 088 505 A1 beschreibt eine Konnektor-Baugruppe für medizinische Anwendungen, die elektrische Kontakte enthält. Diese Kontakte ermöglichen es, eine elektrische Verbindung herzustellen, sobald der Konnektor korrekt verbunden ist, und steuern externe medizinische Geräte durch die Aktivierung von Signalleitungen.

Das Dokument US 2016/0073929 A1 stellt einen Gasprobenstecker vor, der elektronische Komponenten wie Speicher und Daten-/Stromschnittstellen enthält. Der Stecker verbindet sich mit einem Gassampling-Gerät, speichert Informationen wie Nutzungsdauer und Anzahl derVerbindungen, und ermöglicht die Authentifizierung und Nachverfolgung. Daten werden über elektrische Verbindungen zwischen dem Stecker und dem Gerät ausgetauscht.

### DAS DOKUMENT US 2016/0114104A1 BESCHREIBT EIN MEDIKAMENTEN-INFUSIONSSYSTEM, DAS ELEKTRONISCHE UND OPTISCHE KOMPONENTEN ZUR SICHEREN VERABREICHUNG VON MEDIKAMENTEN VERWENDET. ES UMFASST IN LEITUNGEN EINGEBETTETE ELEKTRISCHE LEITUNGEN UND OPTISCHE WELLENLEITER, DIE MEDIKATIONSDATEN ÜBERTRAGEN. EIN DRAHTLOSER TAG UND EIN CONTROLLER ÜBERWACHEN DEN VERBINDUNGSSTATUS ZWISCHEN MEDIKAMENTENQUELLE UND PATIENT UND NUTZEN RFID-TAGS, OPTISCHE SENDER UND EMPFÄNGER SOWIE MIKROPROZESSOREN ZUR STEUERUNG DES MEDIKAMENTENFLUSSES UND ZUR SICHERSTELLUNG DER RICHTIGEN MEDIKATION BEIM PATIENTEN.AUFGABE UND LÖSUNG

Aufgabe der Erfindung ist es, ein Fluidleit- und -transfersystem bereitzustellen, welches die genannten Defizite nicht oder nur in geringerem Maße aufweist und darüber hinaus vorzugsweise folgende funktionellen Anforderungen erfüllt: 1. Das Fluidleit- und -transfersystem sollte im Idealfalle seine Integrität von der Fluidquelle, also vom Medikamentenbehälter, Infusionsbeutel, Medikamentenfläschlein oder dergleichen bis zum Patienten selbstständig validieren, Fehlkonnektionen und Medikamenteninkompatibilitäten erkennen und die Therapie validiert dokumentieren können. Unter Validierung soll hier verstanden werden, dass ein Medikament nicht nur z.B. die Infusionspumpe passiert hat, sondern auch in einen IV-Katheter gelangt ist. 2. Sowohl die IV-Therapie als ubiquitäre und ressourcenintensive Therapie als auch die Anwendung von Transfusionssystemen, Dialysesystemen, Ernährungssystemen, Drainagesystemen, Herz-Lungen-Maschinen, extrakorporalen Kreislaufunterstützungssystemen, Systemen zum extrakorporalen Gasaustausch oder Beatmungssystemen sollten sich in moderne, elektronische Arbeitsabläufe eingliedern lassen. D. h. das voraussichtliche Therapieende, notwendige Spritzenwechsel, Ernährungslösungswechsel, Drainagewechsel usw. müssen vom Fluidleit- und -transfersystem selbständig gemeldet und angefordert werden. 3. Das Fluidleit- und -transfersystem muss so robust sein, dass eine Systemtestung zum Beispiel die Durchführung häuslicher Infusions- und Chemotherapien erlauben kann.

Weiterhin ist Aufgabe der Erfindung, für ein solches Fluidleit- und -transfersystem geeignete Komponenten bereitzustellen.

Die Aufgabe der Erfindung wird primär durch ein Anschlussstück zur fluidischen Kopplung medizinischer Geräte und/oder medizinischer Leitungen gemäß unabhängigem Anspruch 1 gelöst.

Ein Anschlussstück im Sinne der Erfindung ist eine Kopplungseinrichtung am Ende einer Fluidleitung oder als Eingang oder Ausgang eines medizinischen Geräts, die mit einem gegensätzlichen Gegen-anschlussstück oder mit einem identischen Anschlussstück mechanischen gekoppelt werden kann, um die Anschlussstücke in die Lage für Fluidaustausch zu versetzen. Die Erfindung betrifft dabei besondere Anschlussstücke aus dem Bereich der Medizin, die nachfolgend über die zugrundeliegende Norm näher spezifiziert sind. Erfindungsgemäße Anschlussstücke können mit normgerechten Gegen-Anschlussstücken unter Bildung einer Fluidverbindung gekoppelt werden.

Konkret betrifft die Erfindung die folgenden Anschlussstücke:
- Verbindungsstücke gemäß der Normenfamilie ISO 80369, insbesondere Verbindungsstück für intravaskuläre oder hypodermische Anwendungen ISO 80369-7, Verbindungsstück für enterale Anwendungen gemäß ISO 80369-3 und Verbindungsstücke für neuroaxiale Anwendungen gemäß ISO 80369-6,
- Stopfen (für eine Infusionsflasche gemäß ISO 8536-2, Bördelkappen für eine Infusionsflasche gemäß ISO 8536-3 und Einstechteile gemäß ISO 8536-4 zum Anschluss an eine Infusionsflaschen oder einen Beutel,
- subkutan implantierte Ports gemäß ISO 10555-6 und stanzfreie Kanüle zur Ankopplung an einen solchen implantierten Port gemäß ISO 10555-6,
- konische Konnektoren für Anästhesie- und Beatmungsgeräte gemäß ISO 5356-1 und Verbindungsstücke gemäß ISO 5367,
- Anschlussstücke für chirurgische Wunddrainagesysteme gemäß ISO 20697,
- Anschlussstücke eines extrakorporalen Kreislaufs eines Hämodialysationssystems, Hämodiafiltrationsystems oder Hämofiltersystems als Blutport gemäß ISO 8637-1 (Abschnitt 4.4.3, Bild 1 der ISO Norm) und Anschlussstücke zum Anschluss an Blutports für Dialysesysteme gemäß ISO 8637-2 (Bild 1 der ISO Norm) sowie
- Anschlussstücke zur internen Konnektierungeines Dialysesystems als Port für die Dialyseflüssigkeit gemäß ISO 8637-1 (Abschnitt 4.4.4, Bild 2 der ISO Norm).

Die Normenangaben beziehen sich auf die jeweils im Juli 2020 geltenden Normen.

Ein erfindungsgemäßes Anschlussstück gestattet es in üblicher Art und Weise den jeweiligen Anschlusstücken, eine Flüssigkeitskopplungzwischen den Anschlussstücken zu schaffen. Hierfür weist ein erfindungsgemäßes Anschlussstück einen durch einen Leitungsabschnitt umgebenen Fluidkanal zur umfänglich dichten Ankopplung in einer Steckrichtung an das Gegen-Anschlussstück zum nachfolgenden Austausch von Flüssigkeiten und/oderGasen mit dem Gegen-Anschlussstück auf. Erfindungsgemäße Anschlussstücke sind mit normgerechten Anschlussstücken, die nicht erfindungsgemäß ausgebildet sind, bzgl. der Flüssigkeitsleitung kompatibel.

Eine besonders übliche und bevorzugte Form der Flüssigkeitskopplungsiehtvor, dass beidseitig am Anschlussstück und am Gegenanschlussstücke konische Leitungsabschnitte vorgesehen sind, die bei der Kopplung ineinandergeschoben werden und klemmend halten.

Zusätzlich zum Leitungsabschnitt weist ein erfindungsgemäßes Anschlussstück eine Übertragungseinrichtung zur Datenübertragung mit dem Gegen-Anschlussstück auf. Die Übertragungseinrichtung kann zusätzlich zur Übertragung elektrischer Energie ausgebildet sein. Wenngleich grundsätzlich getrennte Übertragungseinrichtungen für Daten und für elektrische Energie möglich sind, handelt es sich bevorzugt um eine kombinierte Übertragungseinrichtung zur Datenübertragung und/oder zur Übertragung elektrischer Energie. Jegliche im Kontext dieser Anmeldung erläuterten Übertragungseinrichtungen sind derart zu verstehen, dass es auch eine kombinierte Übertragungseinrichtung oder eine separate Übertragungseinrichtung für elektrische Energie oder Daten sein kann.

Ein erfindungsgemäßes Anschlussstück gestattet es durch seine Übertragungseinrichtung, im Kontext der genannten Fluidleit- und -transfersysteme Verbindungen zwischen Einzelkomponenten herzustellen, durch die nicht nur ein Fluidaustausch erfolgen kann, sondern weiterhin auch elektrische Leistung und/oder digitale Daten zwischen den Anschlussstücken ausgetauscht werden können.

Der Bedarf der Übertragung elektrischer Energie oder elektrischer Daten ist insbesondere gegeben, um Verbraucher, die im Fluidpfad dem Anschlussstück folgen, mit Energie zu versorgen. Es kann sich jedoch auch um eine Stromleitungsfunktion von einem Anschlussstück zu einem am anderen Ende einer Fluidleitung vorgesehenen zweiten Anschlussstück handeln.

Datenübertragung am Anschlussstück kann dem Zweck dienen, Informationen auszutauschen, insbesondere um die Topologie zu prüfen, um Sensordaten auszutauschen und ggf. abzugleichen oder um anderweitige Steuerfunktionen oder Informationserfassungsfunktionen zu übernehmen. Ein sehr einfacher Anwendungsfall ist gegeben, wenn die Datenübertragung erfolgt, um Produktionsdaten des Anschlussstücks auszulesen wie bspw. die Produktionscharge oder ein Ablaufdatum. Ebenfalls ein recht einfacher Anwendungsfall ist gegeben, wenn ein Anschlussstück dafür ausgebildet ist, an ein Gegen-Anschlussstück Informationen über die zulässige Verwendung des Anschlussstücks zu übermitteln, so dass Fehlkonnektierungen erkannt werden können.

Die Übertragungseinrichtung kann in unterschiedlicher Weise ausgebildet sein, wie im Weiteren erläutertwird. Sie ist jedoch jeweils derart ausgebildet, dass ein Kopplungsvorgang in Hinblick auf die Fluidkanäle des Anschlussstücks und des Gegen-Anschlussstücks gleichzeitig auch zur Kopplung der Daten- und/oder Energieübertragungsvorrichtung führt.

Erfindungsgemäße Anschlussstücke in der Medizintechnik sind üblicherweise überwiegend als Kunststoffteile ausgebildet, die gegebenenfalls Limitationen der jeweiligen Normen einhaltend. Die Übertragungseinrichtung umfasst vorzugsweise metallische oder polymere Leiter, die insbesondere vorzugsweise in den Kunststoff des Anschlussstücks eingebettet sind. Es sind aber auch Gestaltungen möglich, bei denen die Leiter oder hiermit verbundenen Kontaktflächen außenseitig auf den Anschlussstücken angebracht sind.

Erfindungsgemäß weist das Anschlussstück eine Übertragungseinrichtung zur Datenübertragung auf, die derart am Anschlussstück vorgesehen ist, dass sie mit einer korrespondierenden Übertragungseinrichtung am Gegen-Anschlussstück Daten austauschen zuführen kann.

Dabei ist erfindungsgemäß vorgesehen, dass die Übertragungseinrichtung des Anschlussstückes mit mindestens einer Spule zur induktiven Kopplung mit der Übertragungseinrichtung des Gegen-Anschlussstücks ausgebildet ist.

Die induktive Übertragung sieht vor, dass unter Nutzung elektromagnetischer Induktion Signale zwischen zwei gekoppelten Anschlusstücken übertragen werden. Bekannt sind entsprechende Systeme insbesondere durch RFID-Tags und durch den QI-Standard des Wireless Power Consortiums zur Energieübertragung. Die induktive Datenübertragung und Energieübertragung ist vergleichsweise lagetolerant. Dies ist vor allem bei Systemen von Vorteil, die bauartbedingt, beispielsweise durch eine steile Konusgestaltung der Kopplungselemente, keine definierte Endlage der Anschlussstücke gewährleisten können.

Elektromagnetische Induktion hat bei definierter Relativanordnung der Spulen zueinander einen praxistauglichen Wirkungsgrad von über 50%. Auch ein Wirkungsgrad von 80% ist zu erreichen. Dies bedeutet, dass auch bei serieller Anordnung von 5 Paaren von Anschlussstücken noch etwa 30% der Leistung erhalten bleibt.

Die für die induktive Kopplung erforderlichen Spulen sind vorzugsweise sicher vom Fluid getrennt, insbesondere indem sie in einer isolierten Kavität des Anschlussstücks vorgesehen sind. Insbesondere bevorzugt ist es, wenn die Spulen von einem einstückigen Kunststoffteil umgeben sind, das vorzugsweise durch einen Spritzgussprozess bei eingelegter Spule hergestellt ist.

Die Integration der Übertragungseinrichtung in die Anschlussstücke erfolgt derart, dass bei Kopplung zweier Anschlussstücke die Energieübertragung bzw. die Datenübertragung störungsfrei möglich ist. Eine erfindungsgemäß vorgeschlagene Bauweise sieht vor, dass die Übertragungseinrichtung mindestens eine planare Spule aufweist.

Unter einem solchen planaren Element wird ein Element verstanden, welches quer zur Steckrichtung mindestens dreimal so groß ist wie in Steckrichtung. Ein solches planares Element kann insbesondere an oder in einem umlaufenden Kragen angeordnet sein, welcher vorzugsweise aus Kunststoff und/oder einstückig mit dem genannten Lagerabschnitt ausgebildet ist. Im Zuge der Kopplung wird dieser umlaufende Kragen einem anderen umlaufenden Kragen des Gegen-Anschlussstücks angenähert, bis die Spule ausreichend nah ist, um die Datenübertragungzwischen den einander gegenüberliegenden Elementen zu gestatten.

Das planare Element ist daher vorzugweise als den Lagerabschnitt umgebendes Element ausgebildet. Im Falle einer Spule ist diese vorzugweise spiralförmig ausgebildet, also mit variablem Durchmesser.

Eine von der planaren Gestaltung abweichende erfindungsgemäß vorgeschlagene Gestaltung wird jedoch bevorzugt, nämlich eine, bei der die Übertragungseinrichtung eine den Fluidkanal umgebende schraubenförmige Spule aufweist.

Bei dieser Art der Gestaltung ist eine relevante oder sogar primäre Erstreckung der Elemente in Steckrichtung vorgesehen. Die Spule weist vorzugsweise zumindest abschnittsweise eine zylindrische oder konische Formgebung auf, die sich entlang des Fluidkanals erstreckt.

Die Anbringung einer Übertragungseinrichtung dieser Art erfolgt vorzugsweise, indem das Anschlussstück mit einem hülsenförmigen Ringabschnitt versehen ist, an dessen Innenseite, an dessen Außenseite oder innerhalb dessen die schraubenförmige Spule vorgesehen ist.

Dieser hülsenförmige Ringabschnitt ist vorzugsweise einstückig mit dem Leitungsabschnitt ausgebildet. Er kann also beispielsweise durch eine durch einen Zwischenraum vom Leitungsabschnitt getrennte umlaufende Wandung gebildet sein. Diese kann beispielsweise zusätzlich einen Kopplungsabschnitt darstellen, an dessen Innenseite oder an dessen Außenseite eine Kopplungseinrichtung zur mechanischen Kopplung mit dem Gegen-Anschlussstück vorgesehen ist, insbesondere ein Innengewinde, ein Außengewinde, eine Bajonettkulisse oder eine Bajonettnocke zum Eingriff in eine Bajonettkulisse.

Insbesondere kann aber auch vorgesehen sein, dass der Ringabschnitt und der Leitungsabschnitt zumindest abschnittsweise identisch sind. Die Wandungen, die unmittelbar den Fluidkanal begrenzen, sind in diesem Falle auch Träger der für die Übertragung vorgesehenen Elemente. Insbesondere kann es sich je nach Typ des Anschlussstücks um eine abschnittsweise konusförmige Wandung handeln, die die dichte Flüssigkeitskopplungermöglicht. Gerade bei Anschlussstücken mit einer solchen zumindest abschnittsweise konischen Wandung ist die Endlage gekoppelter Anschlussstücke vergleichsweise unbestimmt, so dass die Übertragungseinrichtung im hülsenförmigen Ringabschnitt gegenüber einem umlaufenden Kragen bei der planaren Gestaltung aufgrund der reproduzierbaren Beabstandung und in Steckrichtung ausreichend langen Spulen vorteilhaft sein kann. Bei einer zumindest abschnittsweise konischen Wandung, in der eine Spule vorgesehen ist, kann diese ebenfalls entsprechend konisch oder aber zylindrisch ausgebildet sein. Dabei wird die konische Gestaltung bevorzugt.

Die Nutzung des Leitungsabschnitts als Ringabschnitt, der die Übertragungseinrichtung trägt bzw. umgibt, ist gegenüber der Verwendung eines hiervon getrennten hülsenförmigen Kopplungsabschnitts zur Unterbringung der Übertragungseinrichtung mitunter von Vorteil, da hierdurch ein drehbarer Kopplungsabschnitt, beispielsweise in Art einer Überwurfmutter, verwendbar ist, der als drehbares Element nur mit zusätzlichem Aufwand mit einem Leiter einer Fluidleitung verbindbar wäre.

Das Anschlussstück ist vorzugsweise zumindest teilweise aus Kunststoff gefertigt, wobei insbesondere der genannte Leitungsabschnitt, der umlaufende Kragen bzw. der hülsenförmige Ringabschnitt aus Kunststoff gefertigt sein dürfen. Bevorzugt werden dabei jeweils jene Kunststoffe, die in den genannten Normen als Empfehlungen oder Vorgaben zu finden sind.

Grundsätzlich sind Gestaltungen möglich, bei denen sich unmittelbar an die Übertragungseinrichtung ein elektrischer Verbraucher oder ein integrierter Schaltkreisanschließt, ohne dass eine weitere Übertragung von Energie oder Daten über den Verbraucher oder den integrierten Schaltkreis hinaus erfolgt. Üblicherweise ist es jedoch vorgesehen, dass im Anschlussstück und insbesondere zum Zwecke der Weitergabe von elektrischer Energie oder Daten Leitungen im Anschlussstück vorgesehen sind. Hierbei handelt es sich vorzugsweise um metallische oder polymere Leiter.

Bei einem erfindungsgemäßen Anschlussstück kann mindestens eine Ausgabeeinrichtung vorgesehen sein, vorzugsweise in Form einer optischen Ausgabeeinrichtung wie einer LED. Eine solche Ausgabeeinrichtung kann den Status des Anschlussstücks unmittelbar vor Ort signalisieren. Exemplarisch sei die Möglichkeit genannt, dass die Ausgabeeinrichtung über Leuchten oder über die konkrete Leuchtfarbe zu erkennen gibt, ob eine Verbindung des Anschlussstücks korrekt erfolgt ist. So könnte beispielsweise bei einer längeren Leitung aus mehreren Teilstücken schnell erkannt werden, ob alle Anschlussstücke korrekt verbunden sind. Ist dies nicht der Fall, ist dies anhand des fehlenden Leuchtens der entsprechenden LED oder anhand einer entsprechenden Signalfarbe zu erkennen.

Weiterhin ist bei einem erfindungsgemäßen Anschlussstück vorzugsweise vorgesehen, dass mindestens ein integrierter Schaltkreis umfasst ist. Dieser kann insbesondere in ein Anschlussstück integriert sein. Der integrierte Schaltkreis ist mit der Übertragungseinrichtung eines Anschlussstücks verbunden, um Daten oder elektrische Energie zu empfangen.

Der integrierte Schaltkreis kann in einem besonders einfachen Falle lediglich oder primär Kommunikationsaufgaben übernehmen, insbesondere die Kommunikation mit den Datenübertragungseinrichtungen zweier Anschlussstücke. Er kann jedoch auch komplexere Funktionen übernehmen und insbesondere digitale oder analoge Ausgänge oder Eingänge zu umfassen. Die Eingänge können dabei insbesondere mit einer in das Anschlussstück oder der Fluidleitung integrierte Sensorik verbunden sein, beispielsweise einer Sensorik zur Erfassung des Kopplungszustandes des Anschlussstücks oder einer Sensorik zur Erfassung einer Eigenschaft des durchströmenden Fluids oder seines Volumenstroms. Die Ausgänge können zur Steuerung von Aktoren dienen, wobei auch die genannte Ausgabeeinrichtung, insbesondere in Form einer LED, in diesem Sinne ein Aktor ist.

Die Erfindung betrifft darüber hinaus ein Set aus mindestens zwei Anschlussstücken der beschriebenen Art, die nach einem der genannten Systeme zur mechanischen Kopplung aneinander ausgebildet, also üblicherweise männlich und weiblich ausgebildet sind. Dabei sind die beiden Anschlussstücke derart ausgebildet, dass durch die mechanische Kopplung ihre jeweiligen Fluidkanäle in kommunizierende Verbindung miteinander gebracht werden, und ihre jeweiligen Übertragungseinrichtungen in eine Relativposition zueinander gebracht werden, die eine Datenübertragung oder eine Übertragung von elektrischer Energie ermöglicht.

Ein solches erfindungsgemäßes Set weist üblicherweise nicht die beiden isolierten Anschlussstücke auf, sondern zwei Anschlussstücke, die jeweils Teil einer medizinischen Fluidleitung oder eines medizinischen Geräts sind.

Weiterhin betrifft die Erfindung eine medizinische Fluidleitung zum Transport von Flüssigkeiten. Diese Fluidleitung weist einen Leitungskörper mit länglicher Formgebung auf, der von einem Fluidkanal für das Fluid oder Fluidgemisch durchzogen ist. Dabei kann es sich insbesondere um einen starren Rohrkörper oder einen flexiblen Schlauchkörper handeln. Mindestens an einem proximalen Ende des Leitungskörpers, vorzugsweise jedoch sowohl an einem proximalen als auch einem gegenüberliegenden distalen Ende des Leitungskörpers, ist ein Anschlussstück der oben beschriebenen Art vorgesehen, so dass der Kanal des Leitungskörpers mit dem Fluidkanal des Anschlussstücks bzw. der Anschlussstücke in kommunizierender Verbindung steht. Darüber hinaus sind auch Gestaltungen einer Fluidleitung denkbar, die mehr als zwei Enden aufweist, beispielsweise eine Fluidleitung mit einer Y-Konfiguration. In einem solchen Falle sind entsprechende Anschlussstücke vorzugsweise an allen Enden der Fluidleitung vorgesehen.

Wenn die Fluidleitung mit mindestens zwei Anschlussstücken versehen ist, so können diese identisch sein oder in Art eines männlichen und eines weiblichen Anschlussstücks gegensinnig ausgebildet sein.

Entlang des Leitungskörpers mit dem darin vorgesehenen Fluidkanal ist vorzugsweise auch ein elektrisch leitfähiger Leiter zur Leitung von digitalen Daten und/oder zur Leitung elektrischer Energie vorgesehen. Verschiedene Gestaltungen sind hier denkbar. So können im einfachsten Falle der Leitungskörper und der Leiter lediglich parallel zueinander verlaufen, ohne jedoch miteinander verbunden zu sein. Auch ist es möglich, dass der Leiter zwar getrennt von dem Leitungskörper ausgebildet ist, durch Kopplungselemente oder ein schraubenförmiges Umwickeln um den Leitungskörper jedoch mit diesem entlang der Erstreckung des Leitungskörpers verbunden ist. Als besonders bevorzugt werden jedoch integrierte Lösungen angesehen, bei denen der Leitungskörper und die Leitung, also der elektrisch leitfähige Leiter, fest miteinander verbunden sind, so dass diese sich zumindest nicht werkzeuglos voneinander trennen lassen. Insbesondere können der Leitungskörper und die Leitung hierzu in eine gemeinsame Hülle eingefügt sein. Alternativ kann die elektrische Leitung in eine Hülle eingefügt sein, die unmittelbar den Kanal des Leitungskörpers bildet.

Da ein als Schlauchkörper ausgebildeter Leitungskörper ein recht hohes Maß an Dehnbarkeit aufweist, ist der Leiter vorzugsweise derart ausgebildet und/oder geformt, dass er durch eine Dehnung oder Verformung des Leitungskörpers nicht beschädigt wird. Dies kann beispielsweise durch einen polymeren Leiter erreicht werden. Auch ist es möglich, durch die Anordnung des Leiters eine Art Reserve zu schaffen, die eine Längung des Schlauchkörpers unter Nutzung der Leiterreserve ermöglicht. Vorzugsweise ist der Leiter im Bereich des Schlauchkörpers daher mindestens 10% länger als der Schlauchkörper selbst, insbesondere vorzugsweise sogar 50% länger. Der Leiter kann den Fluidkanal des Schlauchkörpers beispielsweise schraubenförmig umgebend ausgebildet sein. Ein länger ausgebildeter Leiter ist auch von Vorteil, wenn der Schlauchkörper als Teil einer Peristaltikpumpe bestimmungsgemäß wiederholt zusammendrückt und entspannt wird. In einem solchen Falle ist es jedoch insbesondere von Vorteil, den Schlauchkörper abschnittsweise ohne Leiter auszugestalten, wie im Weiteren noch erläutert ist.

Die übliche Art der Herstellung sieht vor, dass ein erfindungsgemäßes Anschlussstück als diskretes Anschlussstück genutzt wird, welches mit dem Leitungskörper verbunden wird, beispielsweise angespritzt wird. Es ist aber auch eine Gestaltung möglich, bei der das proximale Ende oder das distale Ende des Leitungskörpers einstückig den Leitungsabschnitt mindestens eines Anschlussstücks bildet. Hierbei ist demnach vorgesehen, dass das Ende des Leitungskörpers unmittelbar den Leitungsabschnitt bildet und dass dabei vorzugsweise die Übertragungseinrichtung ebenfalls unmittelbar am Leitungskörper vorgesehen ist, insbesondere in dessen Wandung integriert ist.

Üblicherweise ist vorgesehen, dass die Einkopplungvon Energie und/oder die Datenübertragung im Bereich eines Anschlussstücks der Fluidleitung erfolgt. Es ist aber auch möglich, eine erfindungsgemäße Fluidleitung in einem Mittelbereich und damit insbesondere beabstandet zu endseitig vorgesehenen Anschlussstücken mit einer hierfür vorgesehenen Anschlusseinrichtung zu versehen. Dies ist von besonderem Nutzen im Zusammenhang mit der im Weiteren noch beschriebenen Nutzung einer Pumpeinrichtung als Master eines Datensystems. Solche Pumpeinrichtungen sind häufig als Peristaltikpumpen ausgebildet und dafür vorgesehen, Leitungskörper einzulegen, die zum Zwecke der Flüssigkeitsförderung von außen mechanische verformt werden. Ist im beschriebenen Mittelbereich mindestens eine Anschlussvorrichtung vorgesehen, so kann diese nach Einlegen des Leitungskörpers von außen mit Daten und/oder elektrischer Energie versorgt werden.

Hierfür wird es als besonders vorteilhaft angesehen, wenn die mindestens eine Anschlusseinrichtung zur Einkopplung/Auskopplung von elektrischer Energie oder von Daten mit einem sich radial vom Leitungskörper erstreckendem Träger versehen, in dem oder an dem die eigentliche Anschlussvorrichtung vorgesehen ist, also beispielsweise eine Spule. Eine solche Spule ist weiterhin vorzugsweise exzentrisch zum Fluidkanal angeordnet, so dass sie den Fluidkanal also nicht umgibt, sondern seitlich versetzt zu diesem angeordnet ist. So kann sie besonders vorteilhaft zur Energieübertragung oder Datenübertragung mit einer Gegenspule genutzt werden, die in der Pumpeinrichtung hierfür vorgesehen ist.

Während bei erfindungsgemäßen Anschlussstücken erfindungsgemäß eine induktive Übertragungseinrichtung vorgesehen ist, um Kontakt zwischen stromführenden Teilen und dem Fluid zu vermeiden, wird es bei der beschriebenen Anschlusseinrichtung im Mittelbereich als plausible Möglichkeit angesehen, eine galvanische Kopplung vorzusehen. Es sind dann an der Außenseite des Schlauchkörpers, insbesondere an dem beschriebenen radial erstreckten Träger, leitfähige Kopplungsflächen vorgesehen, die nach Einlegen der Fluidleitung in die Pumpeinrichtung mit dortigen korrespondierenden Kontaktflächen in Kontakt stehen.

Wird der Schlauchkörper der Fluidleitung bestimmungsgemäß während des Pumpens durch eine Pumpeinrichtung zusammengedrückt, so kann dies schnell zu einer Beschädigung des Leiters entlang der Fluidleitung führen. Um dies zu vermeiden, kann eine erfindungsgemäße Fluidleitung in einem Teilabschnitt in einem Mittelbereich, also zwischen einander gegenüberliegenden Enden der Fluidleitung und dortigen Anschlussstücken, ohne einen Leiter ausgebildet sein. Dieser Teilabschnitt kann dann ohne Beschädigung des Leiters zum Zwecke des Pumpens wiederholt zusammengedrückt werden.

Wenn an beiden Enden der Fluidleitung erfindungsgemäße Anschlussstücke mit einer Übertragungseinrichtung vorgesehen sind, so weist die Fluidleitung vorzugsweise zwei Anschlusseinrichtungen der beschriebenen Art im Mittelbereich auf, nämlich beidseitig des für das Pumpen vorgesehenen Teilabschnitts.

Wie auch bei einem erfindungsgemäßen Anschlussstück kann auch bei einer erfindungsgemäßen Fluidleitung mindestens eine Ausgabeeinrichtung vorgesehen sein. Vorzugweise ist eine solche Ausgabeeinrichtung an einem Anschlussstück oder an zwei Anschlussstücken an gegenüberliegenden Enden der Fluidleitung vorgesehen.

Weiterhin kann auch bei einer erfindungsgemäßen Fluidleitung vorgesehen sein, dass mindestens ein integrierter Schaltkreis umfasst ist, der insbesondere vorzugsweise in ein Anschlussstück der Fluidleitung integriert sein kann. Bei Fluidleitungen mit zwei oder mehr Anschlussstücken ist dennoch vorzugsweise nur eines der Anschlussstücke mit einem integrierten Schaltkreis versehen. Die anderen Anschlussstücke sind dann über Leiter entlang des Leitungskörpers ebenfalls mit diesem integrierten Schaltkreis verbunden. Allerdings sind auch Gestaltungen möglich, bei denen unterschiedliche Anschlussstücke einer Fluidleitung oder auch eines medizinischen Geräts separater integrierte Schaltkreise aufweisen können, die miteinander nicht zur Datenübertragung verbunden zu sein brauchen. So könnte beispielsweise vorgesehen sein, dass zwei unterschiedliche Anschlussstücke einer Fluidleitung oder auch eines medizinischen Geräts jeweils zur Übertragung einer Kennung mittels separater integrierter Schaltkreise ausgebildet sind, wobei diese Kennungen es einem angeschlossenen Gegen-Anschlussstück gestatten, zu erkennen, ob die Kopplung der Anschlussstücke zulässig oder unzulässig ist.

Der integrierte Schaltkreis ist mit der Übertragungseinrichtung eines Anschlussstücks verbunden, um Daten oder elektrische Energie zu empfangen. Er kann in einem besonders einfachen Falle lediglich oder primär Kommunikationsaufgaben übernehmen, insbesondere die Kommunikation mit den Datenübertragungseinrichtungen zweier Anschlussstücke bzw. mit daran angeschlossene Komponenten. Er kann jedoch auch komplexere Funktionen übernehmen und insbesondere digitale oder analoge Ausgänge oder Eingänge umfassen. Die Eingänge können dabei insbesondere mit einer in das Anschlussstück oder die Fluidleitung integrierten Sensorik verbunden sein, beispielsweise einer Sensorik zur Erfassung des Kopplungszustandes des Anschlussstücks oder einer Sensorik zur Erfassung einer Eigenschaft des durchströmenden Fluids oder seinesVolumenstroms. Die Ausgänge können zur Steuerung von Aktoren dienen, wobei auch die genannte Ausgabeeinrichtung, insbesondere in Form einer LED, in diesem Sinne ein Aktor ist.

Die Erfindung betrifft weiterhin ein fluidführendes medizinisches Gerät, welches zur Annahme, Durchleitung und/oder Abgabe von Fluiden und darüber hinaus zur Annahme, Durchleitung und/oder Abgabe von Daten und/oder elektrischer Energie ausgebildet ist. Dieses Gerät weist erfindungsgemäß zur kombinierten Annahme, Durchleitung und/oder Abgabe von Daten und/oder elektrischer Energie sowie Fluid ein Anschlussstück der beschriebenen Gestaltung auf. Im einfachsten Falle wird ein solches Gerät durch eine einfache Fluidleitung der beschriebenen Art gebildet.

Insbesondere sind hiervon aber auch komplexere Geräte umfasst, die der Zwischenspeicherung oder Bereithaltung von Flüssigkeit dienen oder die mindestens einen Aktor zur Steuerung und/oder Druckbeaufschlagung des Fluids aufweisen. Ein solcher Aktor kann beispielsweise ein elektrisch steuerbares Ventil oder eine Pumpe sein. Die Energieversorgung des Aktors kann über die vom Anschlussstück zugeführte elektrische Energie erfolgen oder über eine separate Stromzuführung erfolgen. Steuerdaten zur Steuerung des Geräts können vom Anschlussstück zugeführt werden oder über das Anschlussstück an verbundene Geräte gesendet werden.

Ein Gerät der beschriebenen Art kann weiterhin mindestens einen Sensor aufweisen, dessen Sensordaten vorzugsweise über das Anschlussstück an weitere Geräte übermittelt werden können. Die Energieversorgung kann auch hier über die vom Anschlussstück zugeführte elektrische Energie erfolgen. Der Sensor kann zur Erfassung verschiedener Größen vorgesehen sein. Besonders vorteilhaft ist eine Gestaltung, bei der der Sensor zur Erfassung einer Eigenschaft des Fluids, beispielsweise der Temperatur, oder zur Erfassung des Volumenstroms des Fluids ausgebildet ist. Der Sensor kann jedoch auch zur Erfassung von Umgebungsparametern am Körperoder im Körpereines Patienten vorgesehen sein, beispielsweise als Temperatursensor, Drucksensor oder Sensor zur Erfassung der Sauerstoffsättigung. Ein solcher Sensor kann beispielsweise Teil eines in den Patienten eingebrachten Katheters oder eines implantierten Ports sein.

Vorzugsweise weist das Gerät mindestens einen integrierten Schaltkreis auf, der mit dem Anschlussstück zum Zwecke der Versorgung mit elektrischer Energie oder mit digitalen Daten verbunden ist. Auch dieser integrierte Schaltkreis kann mit Energie betrieben werden, die vom Anschlussstück zugeführt wird und/oder über das Anschlussstück Daten mit anderen Geräten austauschen. Der integrierte Schaltkreis dient insbesondere der Strukturierung der elektronischen Kommunikation mit benachbarten erfindungsgemäßen fluidleitenden Bauteilen. Der integrierte Schaltkreis kann lokal im Gerät die Daten des beschriebenen Sensors verarbeiten und/oder lokal den beschriebenen Aktor steuern. Insbesondere kann der integrierte Schaltkreis genutzt werden, um unter Verwendung des Sensors und des Aktors einen geschlossenen Regelkreis im Gerät zu implementieren. Weiterhin dient der integrierte Schaltkreisinsbesondere der Kommunikation mit über das erfindungsgemäße Anschlussstück verbundene externe Komponenten.

In den meisten Fällen ist es bei einem Gerät mit mindestens zwei erfindungsgemäßen Anschlussstücken zweckmäßig, wenn deren mindestens zwei Übertragungseinrichtung mit einem gemeinsamen integrierten Schaltkreis verbunden sind. Dieser ist vorzugsweise in der Lage, die mindestens zwei Anschlussstücke zu unterscheiden und/oder spezifische unterschiedliche Anschlusskennungen für die beiden Anschlussstücke mittels der Übertragungseinrichtung zu senden. Alternativ ist es jedoch auch möglich, jedes Anschlussstück mit einem eigenen integrierten Schaltkreis zu versehen, welches zum Senden nur einer Anschlusskennung ausgebildet ist, die jedoch ebenfalls für das Anschlussstück spezifisch und eindeutig ist.

Ein erfindungsgemäßes medizinisches Gerät kann in Art sehr unterschiedlicher Geräte realisiert sein.

Das Gerät kann beispielsweise als Fördereinheit ausgebildet sein, worunter zu verstehen ist, dass das Gerät zur Druckbeaufschlagung des Fluids ausgebildet ist. Eine solche Fördereinheit kann insbesondere als Pumpeinrichtung ausgebildet sein und als solche mit einem Kolben ausgestaltet sein. Insbesondere kann es sich jedoch um eine Peristaltik-Pumpeinrichtung handeln, bei der vorgesehen ist, dass ein Fluidschlauch von außen periodisch kraftbeaufschlagt wird, um die Flüssigkeit im Schlauch weiterzubefördern. Eine erfindungsgemäße Fördereinheit mit Peristaltik-Pumpe ist vorzugsweise zum Einlegen einer erfindungsgemäßen Fluidleitung der beschriebenen Art ausgebildet.

Wie auch im Weiteren noch erläutert wird, ist ein erfindungsgemäßes medizinisches Gerät in Art einer Fördereinrichtung und insbesondere ein darin integrierter Schaltkreis auch geeignet, einen Master bei der Datenkommunikation mit an die Fördereinrichtung angeschlossenen weiteren Komponenten zu übernehmen. Ein solcher Master steuert die Kommunikation über die Übertragungseinrichtungen und schickt beispielsweise Steuerbefehle oder Anforderungen zur Datenübermittlungan die anderen Komponenten.

Ein erfindungsgemäßes Gerät kann weiterhin als zentraler Venenkatheter oder peripherer Venenkatheter ausgebildet sein. Ein solcher Katheter bildet einen dünnen Zugang zu einer Vene des Patienten und ist gegebenenfalls für den temporären Verbleib am Patienten vorgesehen. Häufig haben solche Katheter eine Mehrzahl von Anschlussstücken, die die gleichzeitige Zuführung unterschiedlicher Fluide und Fluidgemische ermöglicht. Ein Katheter mit erfindungsgemäßen Anschlussstücken gestattet es, in seinem integrierten Schaltkreis zu hinterlegen, welche Fluidquellen mit ihm verbunden wurden, um auf Basis dessen potentielle Inkompatibilitäten zu erkennen.

Das Gerät kann weiterhin als Mehrwegehahn oder als Hahnbank ausgebildet sein. Ein solcher Mehrwegehahn bzw. eine solche Hahnbank weist mindestens drei Anschlussstücke auf, die vorzugsweise alle als erfindungsgemäße Anschlussstücke ausgebildet sind, und steuert den Fluidstrom im Gerät auf die Anschlussstücke in die Position eines Dreh- und Richtungsventils. Ein erfindungsgemäßer Mehrwegehahn bzw. eine Hahnbank erfasst vorzugsweise die Position mindestens eines Richtungsregulierventils sensorisch und kann mittels seines integrierten Schaltkreises und der erfindungsgemäßen Anschlussstücke die angeschlossenen Geräte identifizieren und seine Ventilposition und Konnektierung erfassen und über die erfindungsgemäßen Anschlussstücke weitergeben, insbesondere an einen oder mehrere Master, die vorzugsweise durch die beschriebenen Fördereinrichtungen gebildet werden.

Das Gerät kann weiterhin als Hämodialysekartusche, als Hämodiafilterkartusche, als Hämofilter oder als Hämokonzentratorkartusche ausgebildet sein. Auf diese Einheiten wird im Rahmen dieser Offenbarung unter dem Begriff Dialysekartusche Bezug genommen.

Das Gerät kann weiterhin als Zusatzporteinrichtung ausgebildet sein und verfügt als solche über eine beidseitig mit einem Anschlussstück versehene Fluidleitung, zwischen denen ein Zusatzport zur fallweisen Hinzufügung eines Fluids vorgesehen ist. Ein solcher Zusatzport bietet die eine temporäre Verbindungsmöglichkeit zum Beispiel zum Zuspritzen von Medikamenten über nadel- oder ventilbasierte Zugangsports am Fluidsystem. Durch die kontinuierliche Energieversorgung des Gerätes kann das Gerät diesen Zuspritzport überwachen. Wenn das Anschlussstück des Zusatzports als erfindungsgemäßes Anschlussstück ausgebildet ist, kann das Gerät zusätzlich mit diesen Zuspritzgeräten, beispielsweise mit einer Spritze mit erfindungsgemäßen Anschlussstück, kommunizieren und so beispielsweise die Art des Medikaments sowie Informationen zur Dosis und zum Hersteller abfragen.

Das Gerät kann weiterhin als Filtergerät ausgebildet sein.

Das Gerät kann weiterhin als Beatmungstubus ausgebildet sein.

Die Aufgabe der Erfindung wird auch durch ein intelligentes medizinisches Fluidleit- und -transfersystem gelöst, welches eine Zugangseinheit zum Applizieren eines Fluids oder eines Fluidgemisches in einen Patienten oder zur Entnahme eines Fluids aus einem Patienten und welches mindestens einen Flüssigkeitsbehälter zur Bereitstellung oder Aufnahme des Fluids aufweist. Dabei finden auch die beschriebenen Anschlussstücke Verwendung, die an der Zugangseinheit und/oder am Flüssigkeitsbehälter und/oder an Fluidleitungen zwischen Flüssigkeitsbehälter und Zugangseinheit verwendet werden.

Das System umfasst vorzugsweise zusätzlich zum Behälter und der Zugangseinheit mindestens ein weiteres Gerät der oben beschriebenen Art, wobei es sich beispielsweise um eine Fördereinrichtung handeln kann.

Ein System in diesem Sinne umfasst somit mindestens die zwingend erforderlichen Komponenten zur Fluidzuführung oder Fluidentnahme, wobei über die Verwendung erfindungsgemäßerAnschlusstücke, insbesondere auch über die Verwendung erfindungsgemäßer Fluidleitungen und/oder Geräte die Grundlage für eine Daten- und/oder Energieübertragung geschaffen ist.

Das System umfasst insbesondere medizinisches Gerät, beispielsweise eine Fluidpumpe, die über einen integrierten Schaltkreis verfügt, der über die vom Anschlussstück geschaffene Datenverbindung Daten des Flüssigkeitsbehälters oder der Zugangseinheit abfragen kann.

Das medizinische Gerät kann im System einen Master bilden, der zur Kommunikation mit anderen Komponenten bzw. den integrierten Schaltkreisen anderer Komponenten des Systems ausgebildet ist, beispielsweise des Flüssigkeitsbehälters oder der Zugangseinheit. Der Master kann jedoch auch von einem erfindungsgemäßen Gerät getrennt ausgebildet sein, also selbst keine unmittelbarfluidführenden Komponenten aufweisen.

Es kann vorgesehen sein, dass ein System mehrere Master hat, beispielsweise mehrere Infusionspumpen, die jeweils bezüglich der ihnen im Flüssigkeitspfad vorgeschalteten und/oder nachgeschalteten Komponenten die Kommunikation mit den jeweiligen integrierten Schaltkreisen der Komponenten steuern. Weist ein System mehrere Master auf, so sind diese vorzugsweise über ein separates Netzwerk, beispielsweise über Ethernet oder einen Feldbus, miteinander verbunden, um Daten ihrer jeweiligen Zweige des Systems auszutauschen oder um Steueraufgaben in den unterschiedlichen Zweigen des Systems miteinander zu koordinieren.

Grundsätzlich können mehrere Master in einem System auch unterschiedliche Aufgaben dahingehend übernehmen, dass ein Master das System mit elektrischer Energie versorgt, während ein anderer Master die Datenkommunikation steuert. Bevorzugt ist es jedoch, wenn eine gemeinsame Mastereinheit sowohl der Energieversorgung der anderen Komponenten als auch der Steuerung der Kommunikation mit diesen anderen Komponenten dient.

Abhängig von der Art des Systems bieten sich unterschiedlich Komponenten bzw. deren integrierte Schalkreise als Master an. Bei einem Infusionssystem ist der Master vorzugsweise durch eine Infusionspumpe des Systems gebildet. Bei einem Dialysesystem ist der Master vorzugsweise durch eine Dialysemaschine des Systems gebildet ist. Bei einem Ernährungssystem ist der Master vorzugsweise durch eine Ernährungspumpe des Systems gebildet. Bei einem Drainagesystem ist der Master vorzugsweise durch eine Drainagepumpe des Systems gebildet. Bei einem Beatmungssystem ist der Master vorzugsweise durch eine Beatmungsmaschine des Systems gebildet.

Weiterhin ist vorzugsweise vorgesehen, dass das System und insbesondere der Master des Systems zur Kommunikation mit einem Server über ein Datennetzwerk ausgebildet sind, insbesondere über ein Intranet oder Ethernet. Dies kann insbesondere geschehen, um zu einem Identifikationscode eines integrierten Schaltkreises Daten abzufragen. So können einem solchen in den integrierten Schaltkreisen des Systems hinterlegten Identifikationscode ergänzende Informationen zugeordnet werden, so beispielsweise ein Haltbarkeitsdatum, eine Chargennummer oder Spezifikationsdaten der Fluidleitung oder des Geräts, in dem der integrierte Schaltkreis vorgesehen ist.

Auch die Kommunikation mit einem Server, um hierdurch die Krankenakte mit Patienten- oder Krankenhausinformationssystem abzufragen oder zu ergänzen, ist zweckmäßig. Die Datenabfrage kann insbesondere dem Zweck dienen, die durch das System bereitgestellte Versorgung des Patienten mit Informationen der Krankenakte abzugleichen. Das Ergänzen der Krankenakte bedeutet, dass die vom System bereitgestellte Versorgung automatisch dokumentiert werden kann.

Die Aufgabe der Erfindung wird auch durch ein intelligentes medizinisches Fluidleit- und -transfersystem gelöst. Dieses System umfasst mindestens eine Fluidquelle zur Bereitstellung eines medizinisch relevanten Fluids oder Fluidgemisches sowie eine Zugangseinheit zum Applizieren des medizinisch relevanten Fluids oder Fluidgemisches in einen Patienten. Das System umfasst weiterhin ein Verbindungsleitersystem, welches die Fluidquelle und die Zugangseinheit verbindet und zum Leiten und Transferieren des medizinisch relevanten Fluids oder Fluidgemisches von der Fluidquelle zum Patienten ausgebildet ist, wobei das Verbindungsleitersystem vorzugsweise mindestens ein regulierendes Transferelement aufweist.

Das intelligente medizinische Fluidleit- und -transfersystem ist vorzugsweise mit mindestens einer der Komponenten gemäß obenstehender Beschreibung ausgerüstet.

Die Komponenten des medizinischen Fluidleit- und -transfersystems sind zumindest zum Teil ausgebildet, Informationen über ihren Statuszu bestimmen und auszutauschen und an einen Masterzu melden. Zur Übertragung dieser Informationen ist das Verbindungsleitersystem dafür ausgebildet, diese Informationen zu übertragen und weiterzuleiten und/oder eine Energieversorgung für die Komponenten des medizinischen Fluidleit- und Transfersystems bereitzustellen, sodass die Komponenten des medizinischen Fluidleit- und -transfersystems eine eigene Informationsinfrastruktur bilden und wobei mittels dieser Informationsinfrastruktureine Netzwerktopologie des Verbindungsleitersystems sowie eines Fluidstromes im medizinischen Fluidleit- und -transfersystem bestimmt werden kann.

Für die Förderung des medizinisch relevanten Fluidgemisches aus der Fluidquelle zur Zugangseinheit am Patienten ist vorzugsweise zwischen der Fluidquelle und der Zugangseinheit eine Fördereinheitzum Fördern des medizinisch relevanten Fluidgemisches ausgebildet. Eine derartige Fördereinheit kann beispielsweise eine Infusionspumpe in einem Infusionssystem sein. In einem Beatmungsgerät erfolgt der Druckaufbau über eine Pumpe oder durch den anliegenden Mediendruck der Beatmungsgase.

In Drainagesituationen für Wundsekret und Urin wird die natürliche Fluidproduktion des Körpers als Antrieb des Fluidstromes genutzt. Bei Drainagesituationen ist der Fluidstrom umgekehrt, d. h. die Fluidquelle liegt im Körper des Patienten und transportiert das Körpersekret in ein außerhalb des Körpers liegendes Reservoir. Der Fluidstrom kann auch über eine Pumpe wie bei einer Thoraxdrainage unterstütztwerden. Die Vorteile der erfindungsgemäßen Informationsinfrastruktur in den Fluidleitsystemen und Verteilelementen kommen hier ebenfalls zum Tragen, da mittels dieser Infrastruktur die Integrität und Topologie des Drainagesystems geprüft werden kann. Als Master kann zum Beispiel die Drainagepumpe fungieren.

In Dialysesystemen, Herz-Lungen-Maschinen oder extrakorporalen Gastaustauschsystemen werden die medizinisch relevanten Fluide oder Fluidgemische über Pumpen oder durch den natürlichen Mediendruck (Blutdruck) angetrieben.

Um die Übertragung von Informationen und Energie zu gewährleisten, sind zumindest in einigen und vorzugsweise in allen fluidleitenden Komponenten des Fluidleit- und -transfersystems, also u. a. in dem Verbindungsleitersystem, metallische oder polymere Leiterbahnen integriert. Hierdurch kann über die Verbindungsleitungen nicht nur die medizinisch relevanten Fluidgemische von einer Fluidquelle zu einer Zugangseinheit in den oder aus dem Patienten erfolgt, sondern parallel dazu Daten, Informationen und /oder Energie zwischen den Komponenten des Fluidleit- und -transfersystems übertragen bzw. transferiert werden.

Unter einem Fluid oder Fluidgemisch werden im Sinne dieser Erfindung Substanzen zusammengefasst, die sowohl gasförmig als auch flüssig sind, ebenso Suspensionen und Aerosole. Unter einem Verbindungsleitersystem im Sinne der Erfindung werden Infusionsschläuche, IV-Katheter, Beatmungsschläuche, Verbindungsschläuche und Verbindungsstücke verstanden.

Über die Informationsinfrastruktur können aktorische Informationen und Befehle zur Veränderung der Strömungstopologie oder informationsanzeigende Inhalte transportiert und transferiert werden. Die Informationsinfrastruktur in dem erfindungsgemäßen Fluidleit- und -transfersystem kann auch innerhalb von medizinischen Geräten - z. B. Infusionspumpen, Dialysemaschinen, Herz-Lungen-Maschinen - oder innerhalb des Patienten, d. h. in der Zugangseinheit lokalisiert sein.

Komponenten des Fluidleit- und -transfersystems, die ausgebildet sind, ihren Status zu bestimmen und an einen Masterzu melden, werden als informationstechnisch aktive Elemente verstanden. Das bedeutet, dass diese Elemente eine Elektronik aufweisen, durch welche die Identifikation (Typ, Seriennummer, Unique Identifier, Lumen-Identifikation bei einem mehrlumigen Katheter), der Zustand (Stellung von Dreiwegehähnen, Katheterklemme geöffnet, Füllstand von Infusionsbehältern), die Eigenschaften (Medikament im Lumen, Herstellungsdetails) und Sensordaten aus dem Patienten übertragen werden. Diese Komponenten können mit einer eigenen Stromquelle ausgebildet sein. Beispielsweise kann in einem Infusionssystem die Infusionspumpe über einen Netzanschluss oder mittels Batterie betrieben werden. Die anderen Komponenten können ebenfalls eine Stromquelle aufweisen.

Dadurch, dass die Komponenten ihren Status bestimmen und melden können, sind die Elemente einer IV-Therapie, eines Dialysesystems, eines Ernährungssystems, eines Drainagesystems, einer Herz-Lungen-Maschine, eines extrakorporalen Kreislaufsystems, eines Systems zum extrakorporalen Gasaustausch odereines Beatmungssystems identifizierbar. Damit istz. B. fürjedes Medikament die patientenseitige Netzwerktopologie bestimmbar. Außerdem können die Verbindungen, Mehrweghähne, Hahnbänke usw. über ihren Typ, eine Seriennummer oder eine ihnen eindeutigzugeordnete ID eindeutig identifiziert werden. Der Vorteil ist, dass durch die eindeutige Erkennung der Komponenten und damit durch die Bestimmung der Netzwerktopologie beispielsweise pharmazeutische Medikamentenunverträglichkeiten erkannt werden können. Wird das erfindungsgemäße Fluidleit- und -transfersystem in der Ausgestaltung eines Infusionssystems mit einer Medikamenten- und Kompatibilitätsdatenbank verbunden, können somit Medikamenteninstabilitäten und Präzipitationen im Infusionssystem erkannt und verhindert werden. Gleiches gilt auch für Transfusionssysteme, Dialysesysteme, Ernährungssysteme, Drainagesysteme, Herz-Lungen-Maschinen, extrakorporale Kreislaufsysteme, Systeme zum extrakorporalen Gasaustausch und Beatmungssysteme. Die vom jeweiligen System identifizierte Netzwerktopologie kann auch mit einer verordneten Therapietopologie verglichen werden und Alarm geben, wenn eine fehlerhafte Medikamentengabe o. ä. vorliegt.

Ein weiterer Vorteil des erfindungsgemäßen Fluidleit- und -transfersystems ist, dass die wirksamen physischen Verbindungen des Fluidleit- und -transfersystems geprüft werden können und damit die Leckagewahrscheinlichkeit gesenkt werden kann. Ebenso kann in unmittelbarer Patientennähe der Durchfluss gemessen und mit den Parametern einer Fördereinheit, beispielsweise einer Infusionspumpe, verglichen und auf Plausibilität geprüft werden.

Die Komponenten des Fluidleit- und -transfersystems weisen jeweils eine Kopplungseinrichtungzur Ein- und Auskopplung von Informationen und/oder zur Einkopplung von Energie auf. Unter einer Kopplungseinrichtung wird im Sinne der vorliegenden Erfindung eine Verbindungsstelle verstanden, die es ermöglicht, Daten von einer Komponente des Fluidleit- und -transfersystems über das Verbindungsleitersystem an eine weitere Komponente des Fluidleit- und -transfersystems oder einen Master zu übertragen bzw. zu transferieren.

Eine derartige Kopplungseinrichtung bzw. Verbindung ist z. B. so ausgebildet, dass die Kompatibilität zum bekannten Luer-System gewahrt wird. Erfolgen kann die Kopplung beispielsweise über einen Luer-Lock auf Luer-Lock oder einen Luer-Slip auf Luer-Slip oder einen Luer-Slip auf Luer-Lock oder über eine Durchstechverbindungvon einem Infusionsbehälter in Form einer Flasche odereines Beutels zum Verbindungsleiter oder über eine Stechverbindung von einer Portnadel auf einen implementierten Port, wobei die jeweilige Kopplung durch eine Elektronik erweitert ist, die eine Übertragung von Daten und Energie ermöglicht. Auch die regulierenden Transferelemente stellen Kopplungseinrichtungen im Sinne der Erfindung dar. Das gleiche Prinzip findet für die Verbindungen in Ernährungssystemen, Beatmungssystemen, Kreislaufunterstützungssystemen, Oxigenierungssystemen, Dialysesystemen und Drainagesystemen Anwendung, wobei die jeweils anwendungstypischen Verbindungsgeometrien genutzt und um eine Kopplungsfunktionalität für Information und Energie erweitert werden. Dafür werden die aus dem Stand der Technik bekannten Kopplungen und/oderTransferelemente ebenfalls mit einer Elektronik erweitert, die eine Übertragung von Daten und Energie ermöglicht. Ein erweitertes Transferelement mit einer entsprechenden Elektronik zum Einkoppeln von Energie und Information ist vergleichbar mit einem Hub, welcher Energie oder Informationssignale an mehrere Komponenten des Fluidleit- und -transfersystems verteilt und transferiert. Ein Transferelement kann an verschiedenen Stellen des Fluidleit- und -transfersystems ausgebildet sein, je nachdem wo die Notwendigkeit besteht, das zu applizierende medizinisch relevante Fluidgemisch zu regulieren.

Die Kopplungseinrichtungen werden auch als Knotenpunkte in der Netzwerktopologie des Fluidleit- und -transfersystems verstanden. An jedem Knotenpunkt des Netzwerkes wird das Signal aufbereitet und/oder geschaltet. Damit ist eine Enumeration der Topologie - ähnlich zum Beispiel einem USB-Baum - und ein Routing von Datenpaketen zu adressierbaren Knoten möglich. Durch schematisch geplantes zeitversetztes Abfragen einzelner Komponenten (Zeitschlitzverfahren) oder mittels Frequenzmultiplex-Verfahren o. ä. wird damit auch zum einen der Energiebedarf reduziert und zum anderen die Kollision von Datenpaketen verhindert.

Die Informationsübertragung und -weiterleitung und/oder Energieversorgung zwischen den Komponenten des Fluidleit- und -transfersystems und dem Verbindungsleitersystem über die Kopplungseinrichtung kann elektrisch leitergebunden in einem Frequenzbereich größer als 100 kHz erfolgen. Die Übertragung von Energie und Datenpaketen erfolgt in einem Frequenzbereich, der nicht mit dem Aktionspotential und den Latenzzeiten von Zellen kollidiert bzw. diese negativ beeinflusst. Frequenzbereiche, die für Elektrochirurgie verwendet werden, werden ausgespart, um eine Signalstörungzu vermeiden.

Erfindungsgemäß ist beim erfindungsgemäßen Fluidleit- und -transfersystems vorgesehen, dass die Verbindung kontaktlos durch induktive Kopplung erfolgt. Die Verbindungsstellen der miteinander kommunizierenden Komponenten sind dafür galvanisch getrennt voneinander ausgebildet. Die physische Verbindung der Komponenten erfolgt nach wie vor über das Luer-System, oder die jeweils anwendungsspezifischen medizinisch zweckmäßigen Verbinder. Allerdings sind die zu verbindenden Teile derart ausgebildet, dass die Übertragung durch induktive Kopplung erfolgt, d. h. durch die gegenseitige elektromagnetische Beeinflussung zweier oder mehrerer räumlich benachbarter elektrischer Stromkreise.

Die Informationsübertragung und -weiterleitung und/oder Energieversorgung zwischen den Komponenten des Fluidleit- und -transfersystems und den jeweiligen Verbindungsleitungen des Verbindungsleitersystems kann auch aus einer Kombination der Übertragungsarten elektrisch leitergebunden, induktiv und kapazitiv erfolgen.

Für alle Übertragungsarten gilt, dass nebeneinander liegende Leitungen des Verbindungsleitersystems auch in komplexen Therapiesituationen sich durch deren Auslegung nicht relevant stören dürfen. Zur Daten- und Energieübertragung werden daher modulierte Trägerfrequenzen, ähnlich der gängigen NFC-Technologien, genutzt. Diese werden bezüglich Amplitude und Frequenz passend gewählt.

Im Unterschied zu den informationstechnisch aktiven Elementen werden Komponenten des Fluidleit- und -transfersystems, die lediglich Informationen und Energie über induktive Kopplung übertragen, als informationstechnisch passive Elemente verstanden.

In einer Ausgestaltung des erfindungsgemäßen Fluidleit- und -transfersystems ist der Master durch die Fördereinheit, die Kopplungseinheit oder in einer Cloud gebildet.

Das erfindungsgemäße Fluidleit- und -transfersystem kann mehrere Master aufweisen. Vorteilhaft ist, wenn jede Fördereinheit - z. B. eine Infusionspumpe, und/oder das Verbindungsleitersystem und/oder Transferelemente - ausgebildet ist, Daten des Systems zu empfangen, auszuwerten und entsprechend zu reagieren. In Infusionssystemen ist z. B. bekannt und gängige Praxis, dass exakt eine Infusionspumpe für jedes Medikament bzw. für jede Infusionslösung genutzt wird. Außerdem bietet die Infusionspumpe durch ihren Netz- oder Batteriebetrieb den Vorteil, stets genug Energiereserven für die Kommunikation mit den restlichen Komponenten des Infusionssystems bereitzustellen. Durch die systembedingte Direktionalität der Infusionspumpe ist auch eine eindeutige Kommunikationslogik vorgegeben, welche folgende Schritte umfasst: i) Abfrage in proximaler Richtung darüber, welches Medikament oder welche Infusionslösung der Pumpe zugeordnet ist; mittels Sensorinformationen, die über eine entsprechende Sensorik in den Medikamentenbehältern ermittelt werden, ist es möglich, den Status der Infusionslösung wie Füllstand, Herstellungsdetails, Chargen, Ablaufdatum und Unique ID zu bestimmen. ii) Abfrage in distaler Richtung darüber, welche Transferelemente vorliegen; welcher IV-Zugang, welches Lumen und welcher Patient an das Infusionssystem angeschlossen ist. Sensordaten wie Körpertemperatur und Sauerstoffsättigung können erfasst und ausgewertet werden. Ähnliches gilt für die Ausbildung des Fluidleit- und -transfersystems als Transfusionssystem, Dialysesystem, Ernährungssystem, Drainagesystem, Herz-Lungen-Maschine, extrakorporales Kreislaufsystem, System zum extrakorporalen Gasaustausch oder Beatmungssystem.

Des Weiteren ist es vorteilhaft, wenn der Master ausgebildet ist, einen uni- oder bidirektionalen Informationsaustausch mit einem Patienten- oder Krankenhausinformationssystem durchzuführen.

Damit ist es möglich, dass das Fluidleit- und -transfersystem beispielsweise notwendige Spritzenwechsel oder ein voraussichtliches Therapieende selbstständig anzeigt und anfordern kann und damit das medizinische Personal entlastet.

Neben der direkten Datenverwertung zur Steuerung und Überwachung der Infusions-, Transfusions-, Dialyse-, Ernährungs-, Drainage-, oder Beatmungstherapie können diese somit auch automatisiert in hoher Qualität dokumentiert werden. Damit ist eine vollständige Nachverfolgbarkeit möglich.

Durch das erfindungsgemäße Fluidleit- und -transfersystem kann die Integrität von der Fluidquelle bis zum Patienten durch das System selbstständig validiert werden. Fehlkonnektionen und Medikamenteninkompatibilitäten werden erkannt und die Therapie kann ohne großen personellen Aufwand lückenlos und zuverlässig dokumentiert werden. Mit dem erfindungsgemäßen Fluidleit- und - transfersystem kann dafür gesorgt werden, Krankenhausressourcen zu sparen und die Qualität des Patientenmanagements zu steigern, indem beispielsweise das voraussichtliche Therapieende, notwendige Spritzenwechsel vom Fluidleit- und -transfersystem selbstständig gemeldet und angefordert werden können.

Ein weiterer Vorteil des erfindungsgemäßen Fluidleit- und -transfersystems stellt auch sein modularer Aufbau dar. Das System ist problemlos durch weitere Fördereinheiten, Fluidquellen und Verbindungsleiter im Verbindungsleitersystem erweiterbar, die ohne Schwierigkeiten der Netzwerktopologie und Informationsinfrastruktur hinzugefügt werden können.

Erfindungsgemäß wird ebenfalls ein Computerprogrammprodukt vorgeschlagen, welches Befehle umfasst, die bei der Ausführung des Programms durch einen Computer diesen veranlassen, die Netzwerktopologie des Fluidleit- und -transfersystems zu analysieren, die Integrität des Fluidleit- und -transfersystems zu prüfen und für die Dokumentation in einer Patientenakte anzuwenden. Dieses Computerprogrammprodukt kann über ein Tablet, welches der Arzt oder das medizinische Personal mit sich führt, bedient werden. Auch die Konzeption der Bedienung des Systems kann derart vereinfacht werden, dass es für die Durchführung z. B. häuslicher Infusions- und Chemotherapien geeignet ist.

In einer vorteilhaften Anwendung ist das erfindungsgemäße medizinische Fluidleit- und -transfersystem als ein Messsystem mit einer Fluidsäule zwischen dem Körperinneren eines Patienten und einem extrakorporalen Messsystem für den arteriellen oder venösen Blutdruck ausgebildet. Dabei wird für die intraarterielle Druckmessung eine Flüssigkeitssäule zwischen einem intraarteriellen Katheter im Patienten und einem extrakorporalen Druckmesssystem für die Übertragung des Blutdrucks verwendet. Die erfindungsgemäße Informationsinfrastruktur in den dazu notwendigen Schläuchen, Verbindungsstücken und Dreiwegehähnen erlaubt auch hier eine automatische Integritätsprüfung, die Erkennungvon Fehlkonnektionen und die direkte Meldung von Spülvorgängen im System und der Messeinheit. Damit werden Messfehler während der Spülzyklen oder der Blutentnahme aus dem System vermieden, da der Master diese Ereignisse aus der dynamischen Topologie (wie beispielsweise der Position von Dreiwegehähnen) erkennen und an das Messsystem kommunizieren kann. Dieses Prinzip gilt analog für die invasive Venendruckmessung.

Zusammenfassend stellt das erfindungsgemäße Fluidleit- und -transfersystem eine Informationsinfrastruktur in der Infusions-, Transfusions-, Dialyse-, Ernährungs-, Drainage-, und Beatmungstherapie dar, die es ermöglicht, die Integrität von der Fluidquelle bis zum Patienten selbstständig zu validieren, Fehlkonnektionen und Medikamenteninkompatibilitäten zu erkennen und die Therapie validiert zu dokumentieren. Daherwird esauch als intelligentes Fluidleit- und -transfersystem bezeichnet. Das erfindungsgemäße System bietet volle Konnektivität, wobei es weitgehend keine physischen Kontaktflächen benötigt. Damit ist das System auch ideal sterilisierbar und es entstehen keine neuen Biokompatibilitätsprobleme, da der Kontakt mit den Medikamenten weiterhin über die dafür erprobten Glas- und Polymermaterialien erfolgt. Des Weiteren ist das System vollständig rückwärtskompatibel durch Integration in den Luer-Standard und bietet sich als mittelfristiger Industriestandard für eine "Smarte Fluidleit- und -transfersystemtechnik" an.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 zeigt eine schematische Darstellung einer Netzwerktopologie eines erfindungsgemäßen Fluidleit- und -transfersystems am Beispiel eines Infusionssystems.
Fig. 2 zeigt in Form einer schematischen Darstellung eine Netzwerktopologie des erfindungsgemä-βen Fluidleit- und -transfersystems am Beispiel eines Dialysesystems.
Fig. 3 zeigt in Form einer schematischen Darstellung eine Netzwerktopologie des erfindungsgemä-βen Fluidleit- und -transfersystems am Beispiel eines Wunddrainagesystems.
Fig. 4 zeigt in Form einer schematischen Darstellung eine Netzwerktopologie des erfindungsgemä-βen Fluidleit- und -transfersystems am Beispiel eines Beatmungssystems.
Fig. 5 zeigt zwei Anschlussstücke in Art eines Luer-Locks mit planarer induktiver Übertragungseinrichtung.
Fig. 6zeigetAnschlussstücke in Art von Luer-Kupplungen mit einer planaren Spule.
Fig. 7zeigt Anschlussstücke in Art von Luer-Kupplungen mit einer hülsenförmigen Spule im Bereich eines Leitungsabschnitts.
Fig. 8 zeigt Anschlussstücke in Art von Luer-Kupplungen mit hülsenförmigen Spulen im Bereich einer Kopplungseinrichtungzu mechanischen Kopplung.
Fig. 9A zeigt die Verbindung zwischen einer Bördelkappe eines Infusionsbeutels oder einer Flasche einerseits und einem Infusionsschlauch mit erweiterter Elektronik andererseits mittels induktiver Kopplung.

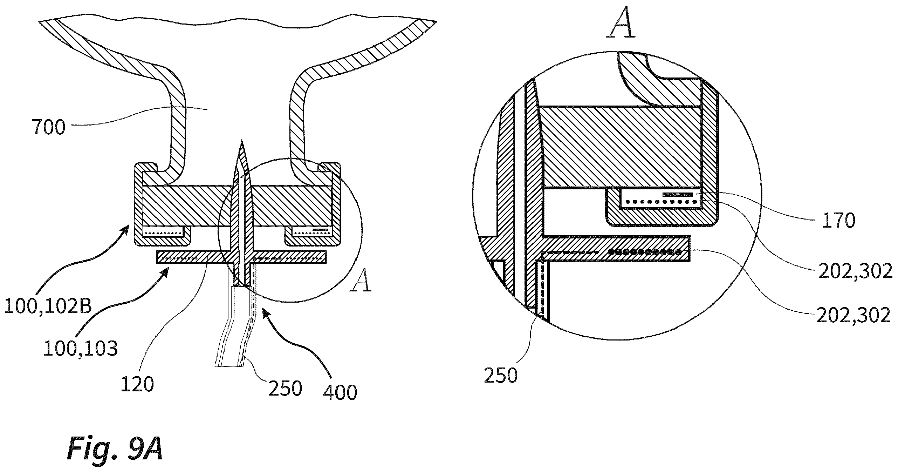

Fig. 9A zeigt die Verbindung zwischen einem Verschlussstopfen eines Infusionsbeutels oder einer Flasche einerseits und einem Infusionsschlauch mit erweiterter Elektronik andererseits mittels induktiver Kopplung.

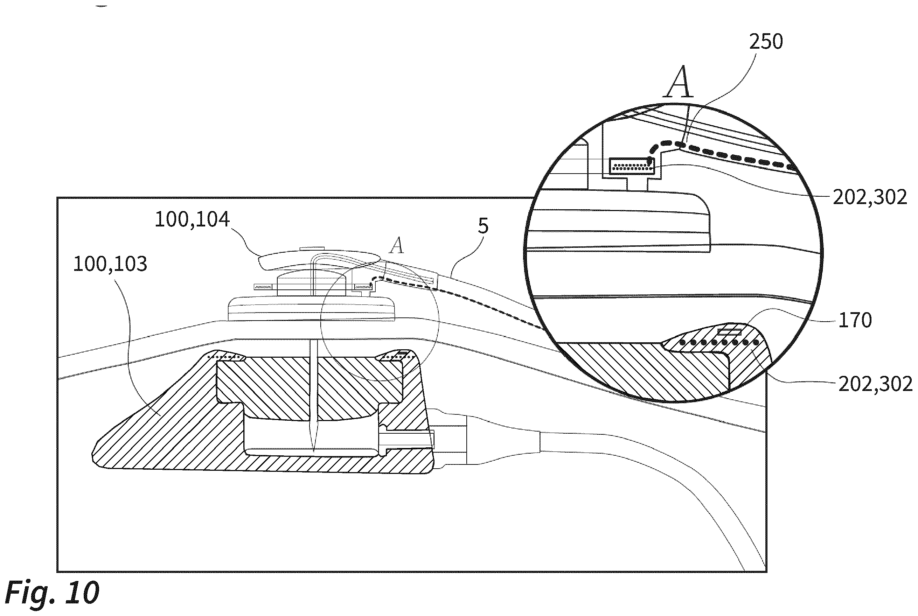

Fig. 10 zeigt die Verbindung zwischen einem implantierten Port und einer Portnadel mit erweiterter Elektronik.

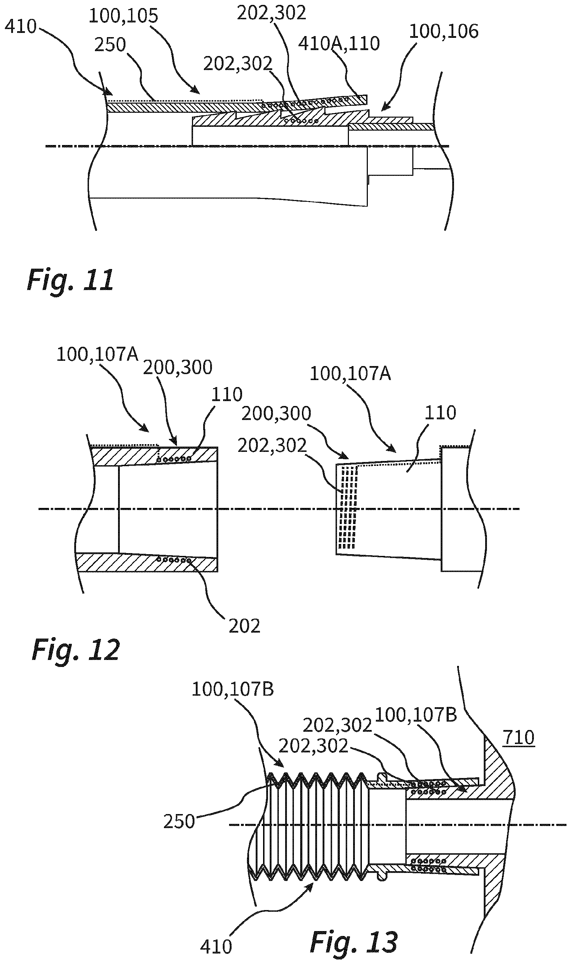

Fig. 11 zeigt ein Anschlussstück, dass durch ein Ende eines Schlauchs gebildet ist, insbesondere als Teil eines Drainagesystems.
Fig. 12 zeigt Anschlussstücke eines Beatmungssystems.
Fig. 13 zeigt Verbindungsstücke eines Beatmungssystems.

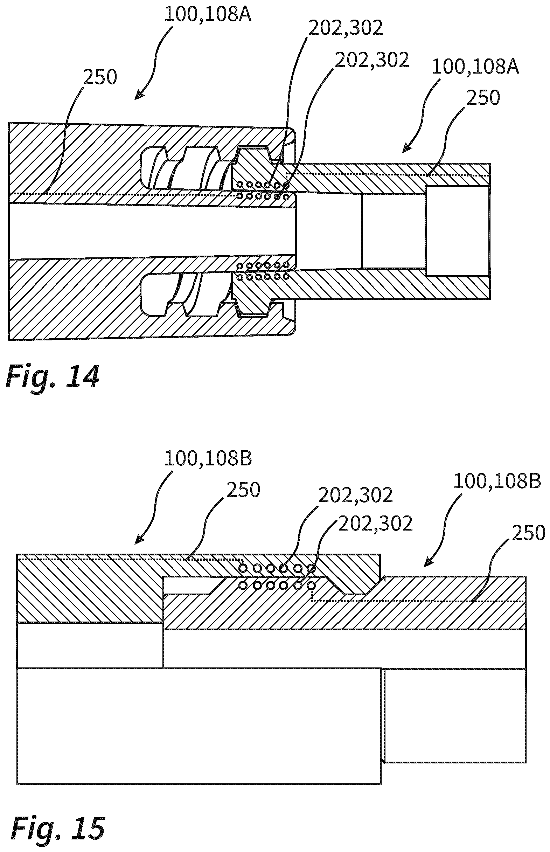

Fig. 14 zeigt Anschlussstücke an Blutports einer Dialysekartusche.
Fig. 15 zeigt Anschlussstücke an Dialysatports einer Dialysekartusche.

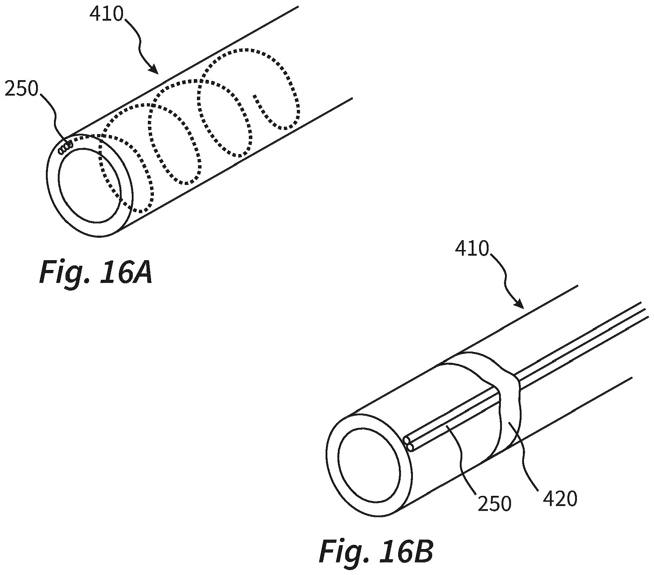

Fig. 16A und 16B zeigen verschiedene Arten der Gestaltung eines Leitungskörpers mit parallel geführtem Leiter.

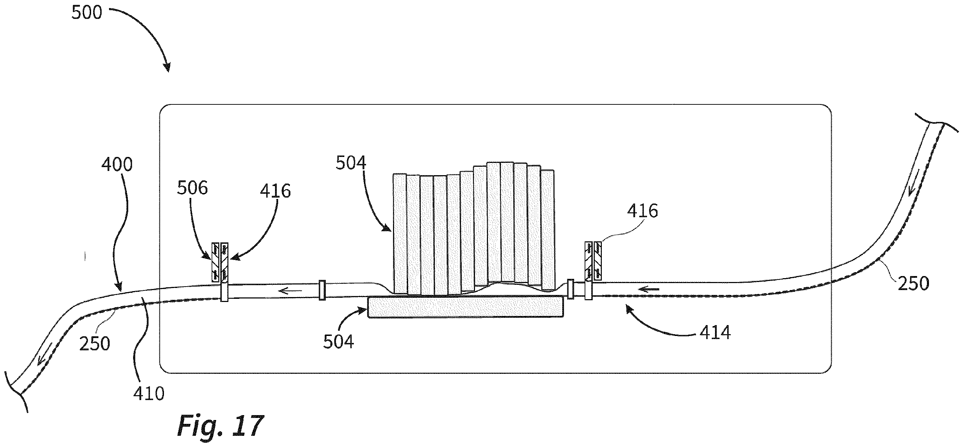

Fig. 17 zeigt eine Infusionspumpe als Master mit Einkopplungvon Energie und Ein- und Auskopplung von Informationen.

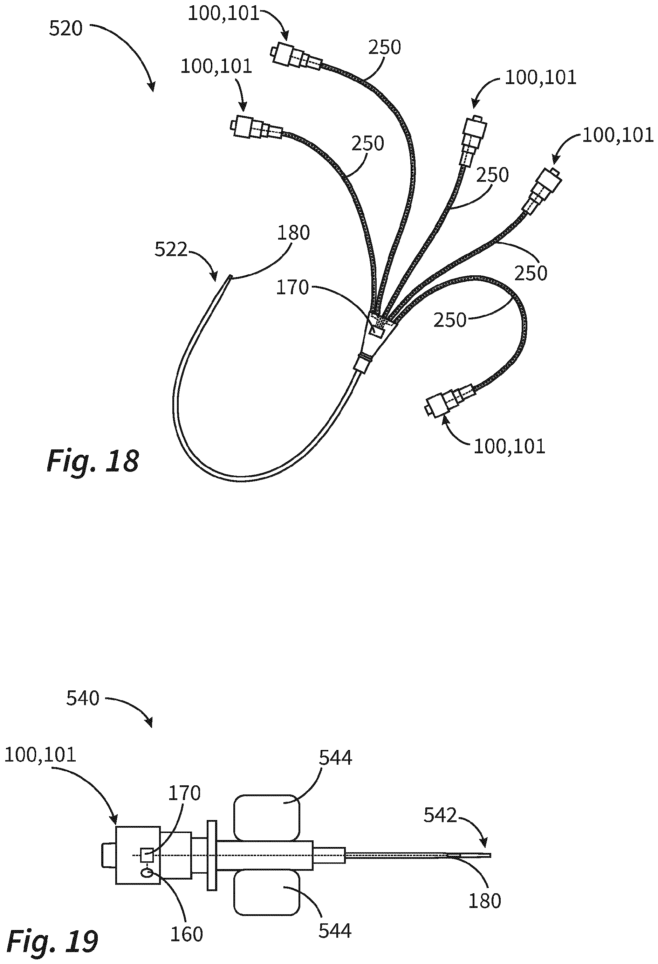

Fig. 18 zeigt einen zentralen Venenkatheter.
Fig. 19 zeigt einen peripheren Katheter.

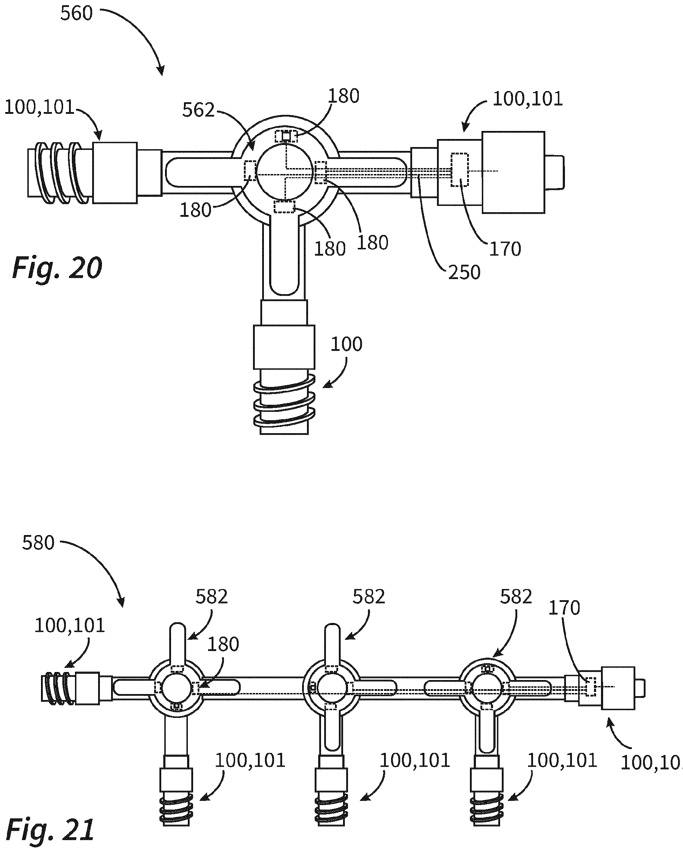

Fig. 20 zeigt einen Dreiwegehahn.
Fig. 21 zeigt eine Hahnbank.

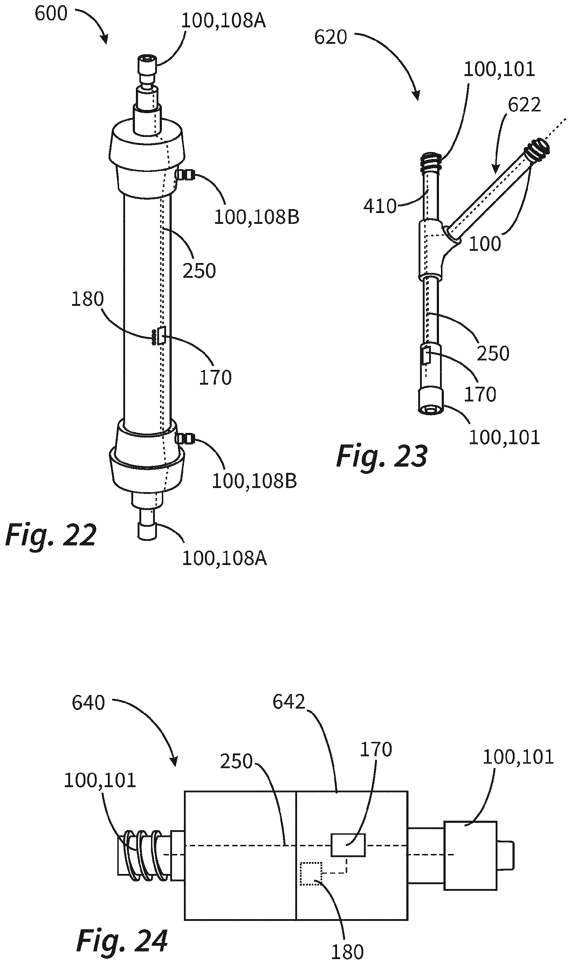

Fig. 22 zeigt eine Dialysekartusche.
Fig. 23 zeigt eine Zusatzporteinrichtung.
Fig. 24 zeigt eine Filtereinheit.

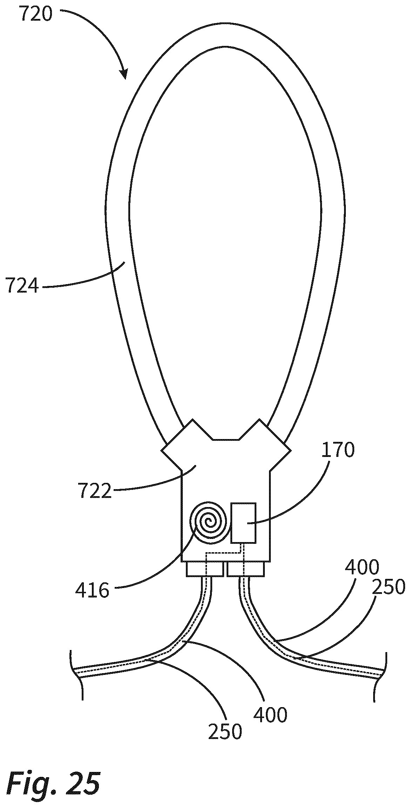

Fig. 25 zeigt eine Schlaucheinheit für eine Peristaltikpumpe.

### DETAILLIERTE BESCHREIBUNG DER AUSFÜHRUNGSBEISPIELE

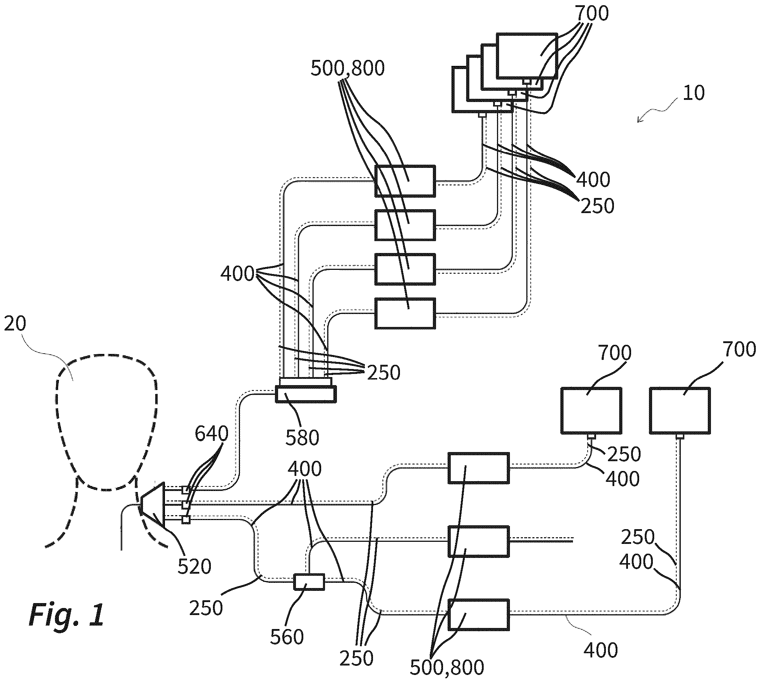

Figur 1 zeigt die schematische Darstellung einer Netzwerktopologie des erfindungsgemäßen Fluidleit- und -transfersystems 10 in der Ausgestaltung eines Infusionssystems. Im Unterschied zu bekannten Infusionssystem-Topologien weisen die Fluidleitungen 400 zwischen den Komponenten 700, 500, 580, 560, 520 des Systems 10 und die fluidführenden Komponenten 700, 500, 580, 560, 520 selbst metallische oder polymere Leiterbahnen 250 auf, die es ermöglichen, Daten/Informationen und/oder Energie weiterzuleiten und auszutauschen. Die Komponenten des Infusionssystems 10 sind jeweils mit einer Elektronik versehen, so dass die einzelnen Komponenten - wie die Transferelemente 560, 580 (z. B. Dreiwegehähne 560 oder eine Hahnbank 580) oder Fluidquellen 700 (wie Infusionsbeutel) oder Fördereinheiten 500 (wie Infusionspumpen) - in der Lage sind, sich zu identifizieren und ihren Status zu bestimmen und zu melden.

Das intelligente Infusionssystem 10 erlaubt beispielhaft die Funktionalität, fehlerhafte Verbindungen im Infusionssystem zu erkennen. Das Gesamtsystem kann mittels erfindungsgemäß integriertem Computercode seine Topologie bestimmen und damit eine Abweichung derverordneten Medikamente und des medizinisch verordneten Therapieschemas von der tatsächlichen Topologie des Infusionssystems feststellen. Diese Überprüfung schließt die Verbindung eines Medikamentes aus einer Fluidquelle 700 zum richtigen Patienten 20 ein. Da die patientenseitigen Komponenten des Infusionssystems - einschließlich zentralem Venenkatheter 520 - im Patienten 20 implantiert sind, erlauben sie eine eindeutige Identifikation des Patienten 20 und damit die Prüfung, ob ein Medikament oder eine Infusionslösung aus einer Fluidquelle 700 - d. h. einem Infusionsbeutel oder einer Infusionsflasche - dem richtigen Patienten 20 verabreicht wird.

Das intelligente Infusionssystem 10 erlaubt beispielhaft die Funktionalität, Inkompatibilitäten zwischen Medikamenten oder zwischen Medikamenten und Infusionslösungen an der Stelle im Infusionssystem 10 zu erkennen, wo die Fluidströme aufeinandertreffen. Dazu greift das Computerprogramm auf Kompatibilitätsdatenbanken zu und vergleicht die vorgefundene Infusionstopologie mit diesen Datenbanken.

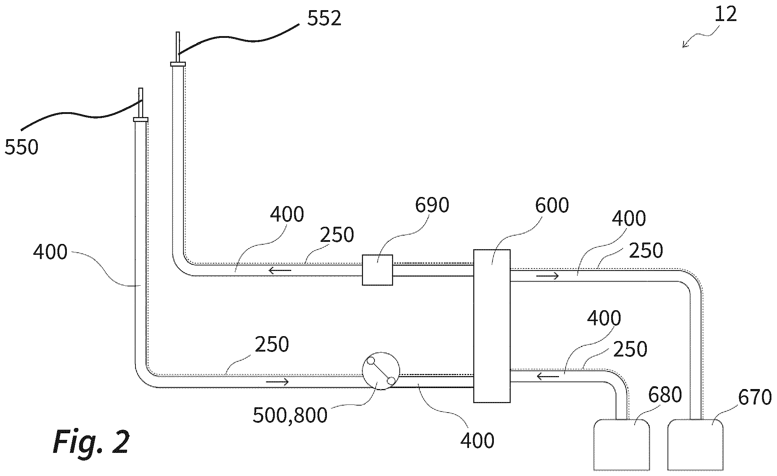

Figur 2 zeigt das erfindungsgemäße Fluidleit- und -transfersystem in der Ausgestaltung eines Dialysesystems 12. Dabei wird eine äquitopologische Informationsinfrastruktur in alle Komponenten des Blutzuflusses - d. h. aus dem venösen Zugang 550 in das Dialysesystem 12 mit der Blutpumpe 500, der Blutleitung 400 zur Blutpumpe 500 und Dialysekartusche 600, den jeweiligen Verbindern (nicht dargestellt) und der Dialysekartusche 600 sowie dem Rückfluss aus Blutleitung 400 zum Patienten, der Luftfalle 690 und dem venösen Zugang 552 - zum Patienten integriert. Auch in allen Komponenten des Dialysesystems 12, wie der Dialyselösung 680 mit der zuführenden Dialyseleitung 400 zur Dialysekartuschen 600, dem Dialysatbehälter 670 mit der Dialysatleitung 400, ist eine Informationsinfrastruktur integriert. Die Verbindungen zwischen den Komponenten werden beispielsweise wie in Figur 2 realisiert. Dadurch können die Integrität dieser Verbindungen zum Patienten und innerhalb der Dialysemaschine verifiziert und Fehlkonnektionen erkannt werden. Als Master 800 und als Energieeinspeisung und Informationsschnittstelle kann beispielsweise die Blutpumpe 500 dienen.

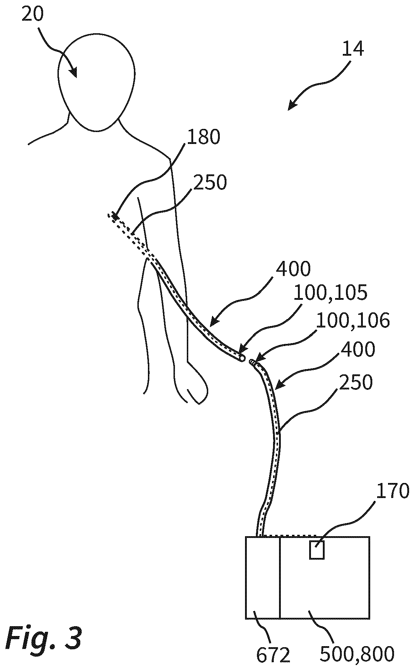

Fig. 3 zeigt das erfindungsgemäße Fluidleit- und -transfersystem in der Ausgestaltung eines Wunddrainagesystems 14. Dieses verfügt über eine Fluidleitung 400, die in den Körper des Patienten 20 geführt ist. Diese in der Figur oben dargestellte Fluidleitung ist mit einer zweiten Fluidleitung 400 verbunden, die mit einer Einheit aus Sekretbehälter 672 und Absaugpumpe 500 verbunden ist. Die Absaugpumpe 500 erzeugt einen Unterdruck im Sekretbehälter 672, wodurch Flüssigkeit durch die Fluidleitungen 400 aus dem Körper des Patienten abgesaugt wird.

Die beiden Fluidleitungen 400 sind als erfindungsgemäße Fluidleitungen ausgebildet und weisen daher Anschlussstücke 105 bzw. 106 auf, die nicht nur ihren Fluidkanal verbinden, sondern auch die an beiden Fluidleitungen 400 vorgesehenen Leiter 250. Auf der Seite des Patienten führt der Leiter 250 bis zum distalen Ende der im Körper des Patienten 20 endenden Fluidleitung 400. Hier ist ein Sensor 180 vorgesehen, der Körperdaten des Patienten 20 erfassen kann. Ein integrierter Schaltkreis 170 der Absaugpumpe 500 ist über die die Leiter 250 und über die Anschlussstücke 100 mit dem Sensor 180 verbunden und kann die Körperdaten somit abfragen und ggf. über eine nicht dargestellte Netzwerkverbindung in einer Patientenakte ablegen.

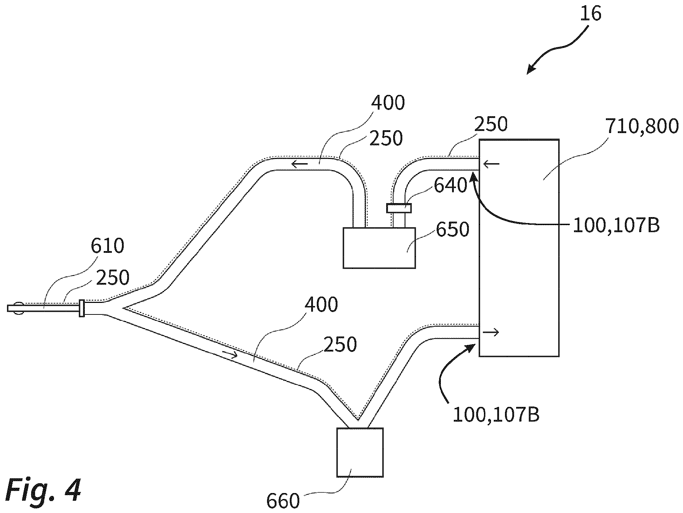

Figur 4 zeigt das erfindungsgemäße Fluidleit- und -transfersystem in der Ausgestaltung eines Beatmungssystems 16. Durch die Leiter 250 in den Beatmungsschläuchen - wie dem Einatmungsschenkel 400, dem Ausatmungsschenkel 400 - besteht eine zusätzliche Prüfmöglichkeit für korrekte Verbindungen, also die richtige Topologie beim Einbau von Befeuchtern 650, Filtern 640 und Flüssigkeitsfallen 660. Die Verbindungen der Informationsinfrastruktur können beispielsweise entsprechend den Figuren 12 und 13 adaptiert auf die Geometrie der Verbindungsstandards im Beatmungssystem induktiv realisiert werden. Sensordaten aus der Beatmungsmaschine 710 oder dem Tubus 610 können über die Informationsinfrastruktur 250 ohne zusätzliche Kabel übertragen werden. Als Master 800 und als Energieeinspeisung und Informationsschnittstelle kann beispielsweise die Beatmungsmaschine 710 dienen.

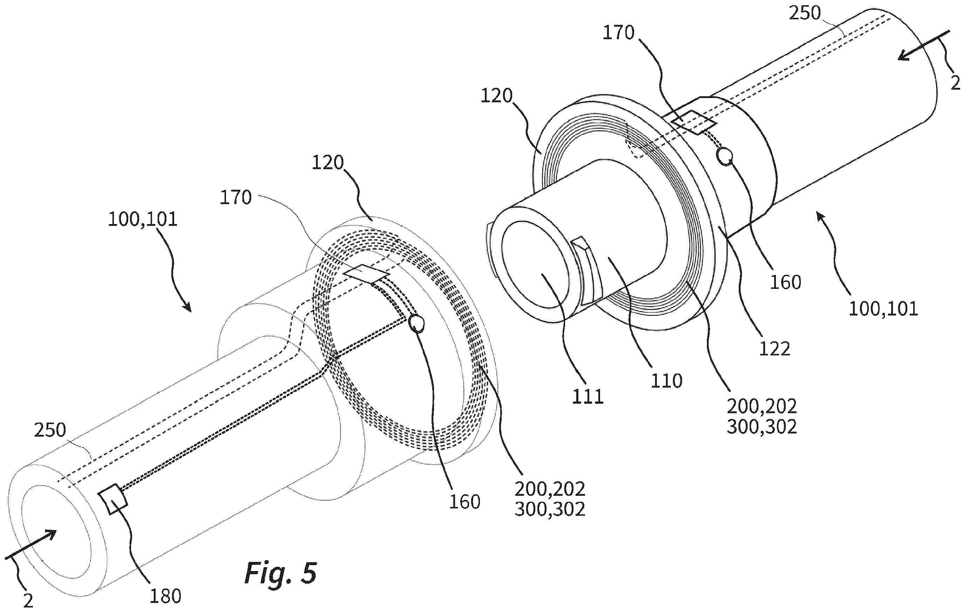

Figur 5 zeigt einen einfachen Luer-Lock gemäß ISO 80369-7, der eine genormte mechanische Verbindung zwischen einem ersten und einem zweiten Anschlussstück 100,101 des Verbindungsleitersystems realisiert. Das erfindungsgemäße Fluidleit- und -transfersystem wahrt die mechanische Kompatibilität zum Luer-System, welches die Verbindung von zwei Teilen durch eine halbe Drehung schließt oder öffnet. Durch die Integration eines Schaltkreises (IC) 170 mit einer Empfängerspule 200, 202 in mindestens einem ersten Anschlussstück 100, 101 der Luer-Lock-Verbindung und einer weiteren Spule 200, 202 in einem zweiten Anschlussstück 100, 101 der Luer-Lock-Verbindung können sowohl Daten als auch Energie übertragen werden. Derartige Anschlussstücke finden bei dem Infusionssystem 10 gemäß Fig. 1 Verwendung.

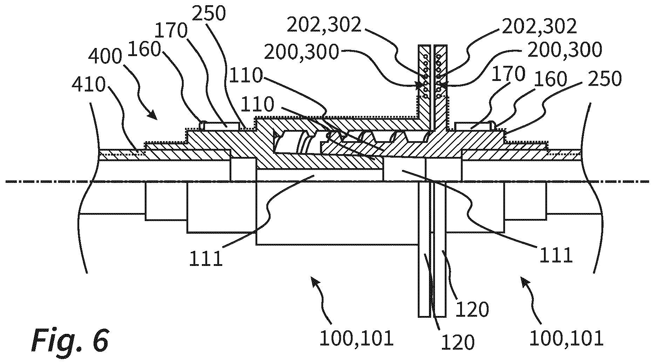

Bei den Anschlussstücken der folgenden Fig. 6 bis 8 handelt es sich vorzugsweise auch um Luer-Lock-Anschlusstücke gemäß ISO 80369-7. Es kann sich jedoch auch um andere Anschlussstücke gemäß der ISO 80369-Normenfamilie handeln.

Fig. 6 zeigt eine Versionen einer Verbindung von zwei Anschlussstücken 100, 101 mit planaren Spulen 202, 302.

Die Anschlussstücke 100, 101 der Fig. 6 entsprechen bezüglich der wesentlichen Merkmale der Gestaltung der Fig. 5. Zwei Fluidleitungen 400 weisen an ihrem Ende jeweils ein Anschlussstück 100, 101 auf, die am Schlauchkörper 410 angebracht ist und jeweils über einen Leitungsabschnitt 110 verfügt, welches von einem Fluidkanal 111 durchdrungen ist. Das in der Figur 6 rechte Anschlussstück 100, 101 ist weiblich und das linke Anschlussstück 100, 101 ist männlich ausgebildet. Dies bedeutet, dass der Leitungsabschnitt 110 des linken Anschlussstücks 100, 101 in den Leitungsabschnitt 110 des rechten Anschlussstücks 100, 101 eingeschoben ist. Die Leitungsabschnitte 110 sind konisch ausgebildet, um eine sichere Fluidverbindung zu schaffen.

Neben der Flüssigkeitskommunikation durch die Leitungsabschnitt 110 sind die Anschlussstücke 100, 101 durch die schon zu Fig. 5 beschriebenen Spulen 202,302 im Bereich von einstückig mit den Leitungsabschnitten 110 ausgebildete Kragen 120 nach Kopplung derart zueinander angeordnet, dass eine Datenübertragung und/oder Energieübertragung möglich ist.

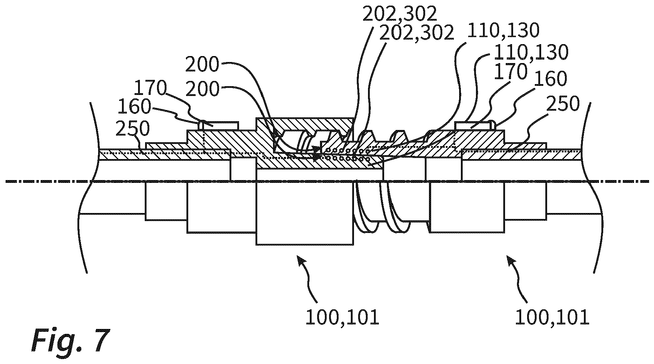

Fig. 7zeigt ebenfalls Anschlussstücke 100, 101 mit konusförmigen Leitungsabschnitten 110. Allerdings sind hier die Elemente zur Daten- oder Energieübertragung, also die Spulen 202, 302, nicht planar ausgebildet, sondern in etwa ring- oder hülsenförmig. Sie sind an oder in Wandungen der Leitungsabschnitte 120 integriert. Hieraus ergibt sich, dass auch bei recht unterschiedlichen Einstecktiefen, wie sie bei Kegelverbindungen möglich sind, eine zuverlässige Übertragung möglich ist. Ein weiterer Vorteil, der sich aus der Nutzung der Leitungsabschnitte als Träger der Übertragungseinrichtungen ergibt, liegt darin, dass drehbare Kopplungsmittel wie Überwurfmuttern verwendet werden können, deren Drehbarkeit es konstruktiv schwierig machen würden, einen Kragen entsprechend den Figur 6 für die Anbringung der Übertragungseinrichtungen zu nutzen.

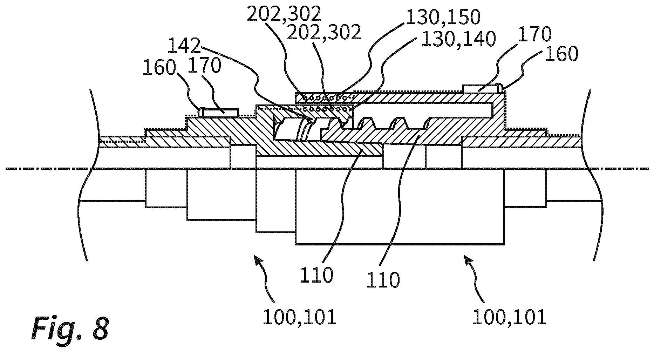

Fig. 8 zeigt nochmals Anschlusstücke 100, 101 mit Spulen, die ring- oder hülsenförmig ausgebildet sind. Allerdings sind die entsprechenden Elemente hier einerseits im Bereich einer Außenseite eines Kopplungsabschnitts 140 bzw. andererseits an einer den Kopplungsabschnitt einhüllenden Mantelwandung 150 vorgesehen.

Figur 9A zeigt eine Verbindung zwischen einem Infusionsbeutel 700 oder einer Infusionsflasche und einem Infusionsschlauch 400 mit erweiterter Elektronik. In dem Infusionsbeutel 700 bzw. sein durch die Bördelkappe 100,102B gemäß ISO 8536-3 gebildetes Anschlussstück 100 ist ein Schaltkreis (IC) 170 mit einer Empfängerspule 202 integriert, wobei in der anzubringenden Infusionsschlauch 400 ein Anschlussstück 103 gemäß ISO 8536-4 mit einer weiteren Spule 202 aufweist, so dass beide Komponenten - wenn sie miteinander verbunden wurden - bezüglich Information und Energie induktiv gekoppelt sind. Damit können Daten und Energie über die integrierte Datenleitung 250 in dem Infusionsschlauch 400 übertragen und ausgetauscht werden.

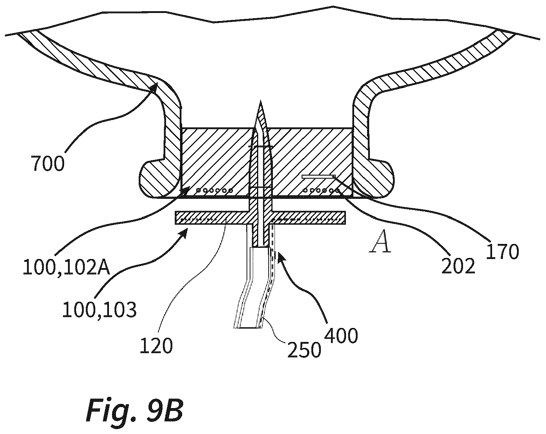

Fig. 9B zeigt eine alternative Gestaltung, bei der das Anschlussstück 100 auf Seiten des Infusionsbeutels 700 oder der Infusionsflasche 700 ein Stopfen 100, 102A gemäß ISO 8536-2 ist, ausgestattet mit einer Übertragungsspule 202 und einem Schaltkreis 170.

Figur 10 zeigt die Verbindung zwischen Anschlussstücken 100 in Art eines implantierten Port 100, 103 und einer Portnadel 100, 104 gemäß ISO 10555-6 und einer Kanüle der Portnadel mit erweiterter Elektronik als Beispiel für eine Zugangseinheit. In dem implantierten Port 100, 103 befindet sich ein Schaltkreis 170 mit einer Empfänger- bzw. Übertragungsspule 202, 302, wobei in der Portnadel 100, 104 eine weitere Empfänger- bzw. Übertragungsspule 202,302 integriert ist, die mit der Datenleitung 25 verbunden ist, die parallel zur Infusionslösung im Infusionsschlauch (d. h. dem Verbindungsleitersystem) ausgebildet ist. Damit ist die Übertragungvon Daten und Energie zwischen Infusionssystem einschließlich der Portnadel 100, 104 und dem implantierten Port 100, 103 möglich.

Fig. 11 zeigt auf der linken Seite einen Leitungskörper 410 einer Fluidleitung 400 in Form eines Schlauchkörpers, dessen Anschlussstück 100 unmittelbarTeil des Schlauchkörpers ist. Die Übertragungseinrichtung des Anschlussstücks ist durch eine hülsenförmige Spule 202 gebildet, die vom Material des Schlauchkörpers umgeben ist. Dieses in den Schlauchkörper integrierte Anschlussstück 100 ist auf ein rechts dargestelltes Gegen-Anschlussstück 100 in Form einer gestuften Klemmhülse vorgesehen. Auch diese ist mit einer hülsenförmigen Spule 202 versehen, welche im dargestellten gekoppelten Zustand gegenüberliegend zur Spule 202 des anderen Anschlussstücks angeordnet ist, so dass eine hohe Energieübertragungseffizienz und eine zuverlässige Datenübertragung erzielt wird. Derartige Anschlussstücke 100 finden bei dem Drainagesystem 14 gemäß ISO 20697 gemäß Fig. 3 Verwendung.

Figur 12 zeigt zwei Anschlussstücke 100, 107A gemäß ISO 5356-1 für ein Beatmungssystem. Ähnlich der Darstellung 7 sind auch hier Übertragungseinrichtungen 200 mit Spulen 202 vorgesehen, die in die konischen Leitungsabschnitte 110 der Anschlussstücke integriert sind.

Fig. 13 zeigt Verbindungsstücke 107B gemäß ISO 5367, die ebenfalls bei einem Beatmungssystem wie dem der Fig. 4 Verwendung finden, insbesondere zum Anschluss von Anschluss einer Fluidleitung 400 mit einem Schlauchkörper 410 an die Beatmungsmaschine 710. Die Verbindungsstücke 107B sind wiederum jeweils mit einer Spule 202 zur Daten- und/oder Energieübertragung ausgebildet. Eines der Verbindungsstücke 107B oder beide Verbindungsstücke 107B können zusätzlich mit einem nicht dargestellten integrierten Schaltkreis versehen sein.

Fig. 14 zeigt zwei Anschlussstücke 108A eines Blutports einer Dialysekartusche 600. Es handelt sich um Anschlussstücke 108A gemäß ISO 8637-1 und ISO 8637-2, die zur dichten Verbindung mittels konischer Leitungsabschnitte ausgebildet sind. Wiederum ist vorgesehen, dass Leiter 250 an den Anschlussstücken 108A vorgesehen sind, die mit Spule 202 zur Daten- und/oder Energieübertragung verbunden sind.

Fig. 15 zeigt zwei Anschlussstücke 108B gemäß ISO 8637-1 eines Dialysatports einer Dialysekartusche 600. Das rechtsseitig dargestellte Anschlussstück ist üblicherweise an der Dialysekartusche 600 vorgesehen. Es weist eine umlaufende Nut auf, in die ein Rastelement des linksseitigen Anschlussstücks 108B einrastet. Auch hier ist vorgesehen, dass Leiter 250 an den Anschlussstücken 108B vorgesehen sind, die mit Spule 202 zur Daten- und/oder Energieübertragung verbunden sind.

Die gezeigten und beschriebenen Anschlussstücke 102A, 102B, 103, 104, 105, 106, 107A, 107B, 108A, 108B können in der in den Fig. 6 bis 8 erläuterten Weise mit Übertragungsvorrichtungen induktiver Art versehen sein sowie die dort beschriebenen Anordnungen (planar, ringförmig im Leitungsabschnitt oder in einem Kopplungsabschnitt) aufweisen.

Fig. 16A und 16B zeigen verschiedenen Gestaltungen des Leitungskörpers 410 von Fluidleitungen 400. Parallel zum Leitungskörper 410 der Fluidleitungen 400 verlaufen jeweils Leiter 250 zur Übertragung elektrischer Energie oderzur Datenübertragung. Bei der Gestaltung gemäß Fig. 16A sind die Leiter in das Material des Leitungskörpers eingebettet. Weiterhin sind die Leiter bei dieser Gestaltung schraubenförmig um den zentrischen Fluidkanal gelegt.

Bei der Gestaltung gemäß Fig. 16B sind ist der Leiter 250 außerhalb des Leitungskörpers 410 angeordnet, mit diesem aber durch umlaufende Kopplungsbänder 420 verbunden.

Figur 17 zeigt eine Infusionspumpe 500, wie sie beim Infusionssystem 10 Verwendung finden kann. Die Infusionspumpe 500 ist als Peristaltikpumpe ausgebildet. Sie weist eine Druckeinrichtung 502 mit einer Mehrzahl von Stempeln auf, die oberhalb einer Gegendruckfläche 504 angeordnet sind. Zwischen der Druckeinrichtung 502 und der Gegendruckfläche 504 ist ein Aufnahmeraum für eine Fluidleitung 400 vorgesehen. Ist die Fluidleitung 400 im Zustand der Fig. 17 eingelegt, so kann Fluid mittels der Druckeinrichtung und deren Stempeln durch die Fluidleitung 400 gefördert werden.

Die Fluidleitung 400 ist mit zwei Leitern 250 versehen, welcher mit in Fig. 17 nicht dargestellten Anschlussstücken an den Leitungsenden stromaufwärts und stromabwärts der Druckeinrichtung 502 verbunden sind. Im Mittelbereich, in dem die Fluidleitung von der Druckeinrichtung kraftbeaufschlagt wird, ist kein Leiter vorgesehen. Stattdessen enden die Leiter 250 anjeweils einer Anschlusseinrichtung 416 in Form einer Spule zur induktiven Übertragung von elektrischer Leistung und Daten, die seitlich am Leitungskörper angebracht ist und in unmittelbarer Nähe zu einer fest in der Fördereinrichtung 500 angeordneten Spule 506 vorgesehen ist.

Auf diese Weise kann die Fördereinrichtung 500 bzw. ein integrierter Schaltkreis der Fördereinrichtung mit weiteren Komponenten des Infusionssystems stromaufwärts und stromabwärts kommunizieren bzw. diese mit elektrischer Leistung versorgen.

Fig. 18 zeigt einen zentraler Venenkatheter 520. Dieser weis einen Zugangsschlauch 522 auf, der durch fünf Eingänge gespeist wird. Diese sind jeweils mit einem erfindungsgemäßen Anschlussstück versehen. Über Leiter 250 kommunizieren alle Anschlussstücke bzw. an diesen ankoppelbare Elemente des Infusionssystems 10 mit einem integrierten Schaltkreis 170. Dieser kann auf diese Daten dazu zusammenführen, welche Fluidquellen an den Anschlussstücken 100 angeschlossen sind. Hierdurch sind problematische Fluidkombinationen oder auch Abweichungen von einem Medikationsplan erkennbar. Möglich ist auch eine Gestaltung, bei der der integrierte Schaltkreis 170 zusätzlich mit einem Sensor 180 kommuniziert, der Sensordaten im Körper des Patienten erfasst.

Der periphere Katheter 540 der Fig. 19 weist ebenfalls einen Zugangsschlauch 542 auf. Dieser wird allerdings nur von einem als Anschlussstück 100 ausgebildeten Zugang gespeist. In der üblichen Form ist der Katheter mit Flügeln 544 zur Befestigung vorgesehen.

Die Übertragungseinrichtung im Anschlussstück 100 ist mit einem integrierten Schaltkreis versehen, welcher wie auch beim Katheter der Fig. 14 mit einem Sensor 180 kommunizieren kann. Zudem ist eine LED 160 vorgesehen, die beispielsweise anzeigen kann, wenn ein vorgegebener Grenzwert durch den Sensordaten überschritten oder unterschritten wird.

Figur 20 zeigt einen Dreiwegehahn, der insgesamt drei Anschlusstücke 100 aufweist
Der Dreiwegehahn verfügt in nicht näher dargestellter Weise über ein Richtungsregulierventil, das manuell mittels eines Griffs 562 verstellt werden kann, um zu steuern, welcher Eingang kommunizierend mit dem Ausgang verbunden ist.

Zur Erfassung des gegenwärtigen Zustandes des Richtungsregulierventil sind Sensoren 180 vorgesehen, beispielsweise Hallsensoren, die die Position eine am Ventil oder am Griff angebrachten Magneten 182 erfassen. Diese Information kann von einem integrierten Schaltkreis 170 an eine verbundene Komponenten, beispielsweise einen als Master agierenden integrierten Schaltkreis der Fördereinrichtung 500 übertragen werden.

Fig. 21 zeigt eine Hahnbank 580, deren Aufbau funktional einer Kopplung mehrere Dreiwegehähne entspricht. Die Hahnbank 580 verfügt über drei Richtungsregulierventile mit Griffen 582, die in gleicher Art und Weise wie der Dreiwegehahn der Fig. 17 mit Sensoren 180 zur Erfassung und Übermittlung des derzeitigen Schaltzustandes mittels eines integrierten Schaltkreises 170.

Fig. 22 zeigt eine Dialysekartusche 600, wie sie im System 12 der Fig. 2 Verwendung findet. Die Dialysekartusche 600 verfügt über einen Anschlussstück 100 am oberen Ende, das für den Einlass des Blutes vorgesehen ist, und ein Anschlussstück 100 am unteren Ende, an dem das Blut abgegeben wird. Die seitlichen Anschlussstücke 100 bilden den Dialysateinfluss und den Dialysatausfluss.

Alle vier Anschlusstücke 100 sind als erfindungsgemäße Anschlussstücke mit einer Datenübertragungseinrichtung ausgebildet und mittels Leitern 250 mit einem außenseitig angebrachten integrierten Schaltkreis 170 verbunden. Dieser kann hierüber mit angeschlossenen Komponenten kommunizieren, um die Validität des Aufbaus zu überprüfen und/oder entsprechende Daten zur Übertragung an einen anderen validierenden integrierten Schaltkreis zu übermitteln.

Fig. 23 zeigt eine Zusatzporteinrichtung 620. Diese umfasst am oberen und am unteren Ende zwei Anschlussstücke 100, die durch eine Schlauchleitung 410 verbunden sind. Zusätzlich ist ein Zusatzport-Abzweig 622 vorgesehen, der vorzugsweise mittels eines Ventils an die Schlauchleitung 410 angeschlossen ist und an dem ein zusätzliches Anschlussstück vorgesehen ist. Hier kann fallweise eine zusätzliche Fluidquelle angeschlossen werden, beispielweise eine Spritze.

Die insgesamt drei Anschlussstücke 100 sind jeweils erfindungsgemäße Anschlussstücke. Sie sind mittels Leitern mit einem integrierten Schaltkreis 170 verbunden, der beispielsweise das Anschlie-βen einer Spritze am Anschlussstück 100 des Zusatzport-Abzweigs 622 registriert und diese Information weiterüberträgt.

Fig. 24 zeigt eine Filtereinheit 640 zur Filtrierung von durchströmendem Fluid. Die Filtereinheit 640 weist ein Filtergehäuse 642 auf, das an gegenüberliegenden Enden über zwei erfindungsgemäße Anschlussstücke 100 als Einlass und Auslass verfügt. Die Anschlussstücke sind über Leiter 250 mit einem integrierten Schaltkreis verbunden. Diese ist zusätzlich noch an einem Filtersensor 180 angeschlossen, der Parameter der Filtrierung erfasst und es dadurch dem integrierten Schaltkreis 170 gestattet, im Falle einer Auffälligkeit die entsprechende Information an eine verbundene Komponente zu übertragen.

Fig. 22 zeigt eine Schlaucheinheit 720 für eine rotatorische Peristaltikpumpe. Eine solche Schlaucheinheit weist eine Schlauchschlaufe 724 auf, die um den Rotor der Peristaltikpumpe gelegt wird. Die beiden Enden der Schlauchschlaufe 724 sind an einem Zentralelement 722 vorgesehen, welches mit einem Zulaufschlauch und einem Ablaufschlauch 400 verbunden sind, die vorzugsweise endseitig über nicht dargestellte erfindungsgemäße Anschlussstücke 100 verfügen.

Am Zentralelement ist neben einem integrierten Schaltkreis 170, der die Kommunikation über die Anschlussstücke steuert, auch eine Anschlusseinrichtung 416 ähnlich jener der Fig. 17 vorgesehen, über den elektrische Leistung und/oder Datenkommunikation für die Schlaucheinheit 720 und damit verbundene Komponenten des Gesamtsystems zur Verfügung gestellt wird.

## Patentansprüche

1. Anschlussstück (100) zur fluidischen Kopplung medizinischer Geräte und/oder medizinischer Leitungen mit den folgenden Merkmalen:
a. das Anschlussstück (100) ist
- in Art eines Verbindungsstücks (101) gemäß der Normenfamilie ISO 80369, insbesondere als Verbindungsstück für intravaskuläre oder hypodermische Anwendungen ISO 80369-7 oder alsVerbindungsstück fürenterale Anwendungen gemäß ISO 80369-3 oder als Verbindungsstücke für neuroaxiale Anwendungen gemäß ISO 80369-6, oder
- in Art eines Stopfens (102A) für eine Infusionsflasche gemäß ISO 8536-2, in Art einer Bördelkappe (102B) für eine Infusionsflasche gemäß ISO 8536-3 oder in Arteines Einstechteils (103) gemäß ISO 8536-4 zum Anschluss an eine Infusionsflaschen oder einen Beutel oder
- in Art eines subkutan implantierte Ports (103) gemäß ISO 10555-6 oder in Art einer stanzfreien Kanüle (104) zur Ankopplung an einen implantierten Port (104) gemäß ISO 10555-6 oder
- in Art eines konischen Konnektors (107A) für Anästhesie- und Beatmungsgeräte gemäß ISO 5356-1 oder in Art eines Verbindungsstücks (107B) gemäß ISO 5367 oder
- in Art eines Anschlussstücks (105, 106) für chirurgische Wunddrainagesysteme gemäß ISO 20697 oder
- in Art eines Anschlussstückes (108A) eines extrakorporalen Kreislaufs eines Hämodialysationssystems, Hämodiafiltrationsystems oder Hämofiltersystems als Blutport gemäß ISO 8637-1 oder in Art eines Anschlussstückes (108A) zum Anschluss an Blutports für Dialysesysteme gemäß ISO 8637-2 oder
- in Art eines Anschlussstückes (108B) zur internen Konnektierung eines Dialysesystems als Port für die Dialyseflüssigkeit gemäß ISO 8637-1
ausgebildet und zur Ankopplung an ein korrespondierend ausgestaltetes Gegen-Anschlussstück (100) ausgebildet, und
b. das Anschlussstück (100) weist einen durch einen Leitungsabschnitt (110) umgebenen Fluidkanal (111) zur umfänglich dichten Ankopplung in einer Steckrichtung (2) an das Gegen-Anschlussstück (100) und zum nachfolgenden Austausch von Flüssigkeiten und/oder Gasen mit dem Gegen-Anschlussstück auf, und
c. das Anschlussstück (100) weist eine Übertragungseinrichtung (200, 300) zur induktiven Datenübertragung mit dem Gegen-Anschlussstück auf,
**gekennzeichnet durch** eines der folgenden zusätzlichen Merkmale:
d. die Übertragungseinrichtung (200, 300) weist mindestens eine planare Spule (202,302) auf, die den Fluidkanal (111) umgebend ausgebildet ist, oder
e. die Übertragungseinrichtung (200, 300) weist eine den Fluidkanal (111) umgebende schraubenförmige Spule (202, 302) auf.

2. Anschlussstück nach Anspruch 1 mit einem der folgenden weiteren Merkmale:
a. die planare Spule (202, 302) ist spiralförmig ausgebildet, oder
b. die schraubenförmige Spule (202, 302) ist zylindrisch oder konisch ausgebildet.

3. Anschlussstück nach Anspruch 1 oder 2 mit dem folgenden weiteren Merkmal:
a. das Anschlussstück (100) weist einen umlaufenden Kragen (120) auf, wobei planare die Spule (202, 302) an oder in dem umlaufenden Kragen (120) vorgesehen ist,
vorzugsweise mit dem folgenden zusätzlichen Merkmal:
b. der umlaufende Kragen (120) ist einstückig mit dem Leitungsabschnitt (110) ausgebildet.

4. Anschlussstück nach Anspruch 1 oder 2 mit dem folgenden weiteren Merkmal:
a. das Anschlussstück weist einen hülsenförmigen Ringabschnitt (130) auf, an dessen Innenseite, an dessen Außenseite oder innerhalb dessen die schraubenförmige Spule (202, 302) vorgesehen ist,
vorzugsweise mit mindestens einem der folgenden zusätzlichen Merkmale:
b. der hülsenförmigen Ringabschnitt (130) ist einstückig mit dem Leitungsabschnitt (110) ausgebildet, wobei vorzugsweise der Ringabschnitt (130) den Leitungsabschnitt (110) zumindest abschnittsweise unmittelbar bildet und wobei vorzugsweise der Ringabschnitt (130) vorzugsweise als zumindest außenseitig abschnittsweise konusförmiger Abschnitt ausgebildet ist, und/oder
c. der hülsenförmige Ringabschnitt (130) bildet einen Kopplungsabschnitt (140), wobei an der Innerseite oder an der Außenseite eine Kopplungseinrichtung (142) vorgesehen ist, insbesondere ein Innengewinde, ein Außengewinde, eine Bajonettkulisse oder eine Bajonettnocke zum Eingriff in eine Bajonettkulisse.

5. Anschlussstück nach einem der vorstehenden Ansprüche mit mindestens einem der folgenden weiteren Merkmale:
a. das Anschlussstück (100) ist zumindest teilweise aus Kunststoff gefertigt, insbesondere der Leitungsabschnitt (110) und/oder der umlaufende Kragen (120) und/oder der hülsenförmige Ringabschnitt (130), und/oder
b. das Anschlussstück (100) weist zur Leitung elektrischer Energie oder zur Leitung von Daten mindestens einen metallischen oder polymeren Leiter (250) auf.

6. Anschlussstück (100) nach einem der vorstehenden Ansprüche mit dem folgenden weiteren Merkmal:
a. das Anschlussstück (100) weist mindestens eine Ausgabeeinrichtung (160) auf, vorzugsweise in Form einer optischen Ausgabeeinrichtung (160) wie einer LED.

7. Anschlussstück (100) nach einem der vorstehenden Ansprüche mit dem folgenden weiteren Merkmal:
a. das Anschlussstück (100) weist einen integrierten Schaltkreis (170) auf, der von der Übertragungseinrichtung (300) mit Strom versorgt wird und/oder mit Übertragungseinrichtung (200) zur Datenübertragung verbunden ist.
vorzugsweise mit mindestens einem der folgenden zusätzlichen Merkmale:
b. der integrierte Schaltkreis (170) weist einen eindeutigen Identifizierungscode auf, und/oder
c. der integrierte Schaltkreis (170) verfügt über mindestens einen digitalen oder analogen Eingang zur Auswertung eines Sensorsignals eines Sensors (180), und/oder
d. der integrierte Schaltkreis verfügt über mindestens einen digitalen oder analogen Ausgang zur Ansteuerung eines Aktors.

8. Anschlussstück (100) nach einem der vorstehenden Ansprüche mit dem folgenden weiteren Merkmal:
a. das Anschlussstück (100) weist einen Sensor (180) auf,
vorzugweise mit mindestens einem der folgenden zusätzlichen Merkmale:
b. der Sensor ist zur Erfassung einer Eigenschaft eines durchströmenden Fluids oder zur Erfassung des Volumenstroms eines durchströmenden Fluids ausgebildet, oder
c. der Sensor ist zur Erfassung eines Kopplungszustandes des Anschlussstücks (100) an einem Gegen-Anschlussstück ausgebildet.

9. Set aus mindestens zwei Anschlusstücken (100) mit den folgenden Merkmalen:
a. die beiden Anschlussstücke (100) sind nach einem der Ansprüche 1 bis 8 und zur mechanischen Kopplung aneinander ausgebildet, und
b. die beiden Anschlussstücke (100) sind derart ausgebildet, dass durch die mechanische Kopplung ihre jeweiligen Fluidkanäle (111) in kommunizierende und umfänglich dichte Verbindung miteinander gebracht werden, und
c. die beiden Anschlussstücke (100) sind derart ausgebildet, dass durch die mechanische Kopplung ihre jeweiligen Übertragungseinrichtungen (200, 300) in eine Relativposition zueinander gebracht werden, die eine Datenübertragung oder eine Übertragung von elektrischer Energie ermöglicht.

10. Medizinische Fluidleitung (400) zum Transport von Flüssigkeiten und/oder Gasen mit den folgenden Merkmalen:
a. die Fluidleitung (400) weist einen Leitungskörper (410) in Form eines starren Rohrkörpers oder eines flexiblen Schlauchkörpers auf, der einen Fluidkanal umgibt, und
b. die Fluidleitung (400) weist an einem proximalen Ende des Leitungskörpers (410) ein Anschlussstück (100) auf, und
c. das Anschlussstück (100) ist nach einem der Ansprüche 1 bis 8 ausgebildet,

11. Medizinische Fluidleitung nach Anspruch 10 mit mindestens einem der folgenden weiteren Merkmale:
a. die Fluidleitung (400) weist an einem distalen Ende ein Anschlussstück nach einem der Ansprüche 1 bis 8 auf, und/oder
b. entlang des Leitungskörpers (410) ist ein elektrisch leitfähiger Leiter (250) zur Leitung von digitalen Daten und/oder zur Leitung elektrischer Energie vorgesehen, und/oder
c. der Leitungskörper (410) weist eine Wandung (412) auf, innerhalb derer der Leiter (250) angeordnet ist.

12. Fluidführendes medizinisches Gerät (100) mit den folgenden Merkmalen:
a. das Gerät ist zur Annahme, Durchleitung und/oder Abgabe von Fluiden ausgebildet, und
b. das Gerät ist zur Annahme, Durchleitung und/oder Abgabe von Daten und/oder elektrischer Energie ausgebildet, und
c. das Gerät weist zur kombinierten Annahme, Durchleitung und/oder Abgabe von Daten und/oder elektrischer Energie sowie eines Fluids ein Anschlussstück nach einem der Ansprüche 1 bis 8 auf.

13. Fluidführendes medizinisches Gerät nach Anspruch 12 mit mindestens einem der folgenden weiteren Merkmale:
a. das Gerät weist mindestens einen Aktor zur Steuerung und/oder Druckbeaufschlagung des Fluids auf, und/oder
b. das Gerät weist mindestens einen Sensor auf, insbesondere einen Sensor zur Erfassung einer Eigenschaft des Fluids oder zur Erfassung des Volumenstroms des Fluids, und/oder
c. das Gerät weist mindestens einen integrierten Schaltkreis auf, der von der Übertragungseinrichtung mit Strom versorgt wird und/oder mit Übertragungseinrichtung zur Datenübertragung verbunden ist.

14. Fluidführendes medizinisches Gerät nach einem der Ansprüche 12 oder 13 mit dem folgenden zusätzlichen Merkmal:
a. das Gerät ist als Fördereinheit (500) ausgebildet, oder
b. das Gerät ist als zentraler Venenkatheter (520) oder als peripherer Venenkatheter (540) ausgebildet, oder
c. das Gerät ist als Hahnbank (580) oder Mehrwegehahn (560) ausgebildet, oder
d. das Gerät ist als Hämodialysekartusche, als Hämodiafilterkartusche, als Hämofilter oder als Hämokonzentratorkartusche (600) ausgebildet, oder
e. das Gerät ist als Zusatzporteinrichtung (620) ausgebildet und verfügt über eine beidseitig mit einem Anschlussstück versehene Fluidleitung, zwischen denen ein Zusatzport zur fallweisen Hinzufügung eines Fluids vorgesehen ist, oder
f. das Gerät ist als Filtergerät (640) ausgebildet, oder
g. das Gerät ist als Beatmungstubus (610) ausgebildet.

15. Medizinisches Fluidleit- und -transfersystem mit den folgenden Merkmalen:
a. das System (10, 12, 14, 16) umfasst eine Zugangseinheit zum Patienten (20) zum Applizieren eines Fluids oder eines Fluidgemisches in einen Patienten (20) und/oder zur Entnahme eines Fluids aus einem Patienten, und
b. dass System umfasst mindestens einen Flüssigkeitsbehälter zur Bereitstellung oder Aufnahme des Fluids auf, und
c. die Zugangseinheit und/oder der Flüssigkeitsbehälter weist ein Anschlussstück gemäß einem der Ansprüche 1 bis 8 auf.

## Claims

1. Connecting piece (100) for fluidic coupling of medical appliances and/or medical lines, having the following features:
a. the connecting piece (100) is
- in the form of a connector (101) of the ISO 80369 family of standards, in particular in the form of a connector for intravascular or hypodermic applications according to ISO 80369-7 or in the form of a connector for enteral applications according to ISO 80369-3 or in the form of connectors for neuraxial applications according to ISO 80369-6, or
- in the form of a plug (102A) for an infusion bottle according to ISO 8536-2, in the form of a crimp cap (102B) for an infusion bottle according to ISO 8536-3 or in the form of a piercing spike (103) according to ISO 8536-4 for connection to an infusion bottle or a bag or
- in the form of a subcutaneous implanted port (103) according to ISO 10555-6 or in the form of a non-coring cannula (104) for coupling to an implanted port (104) according to ISO 10555-6 or
- in the form of a conical connector (107A) for anaesthetic and respiratory equipment according to ISO 5356-1 or in the form of a connector (107B) according to ISO 5367 or
- in the form of a connecting piece (105, 106) for surgical wound drainage systems according to ISO 20697 or
- in the form of a connecting piece (108A) of an extracorporeal circuit of a haemodialysis system, haemodiafiltration system or haemofilter system as a blood port according to ISO 8637-1 or in the form of a connecting piece (108A) for connection to blood ports for dialysis systems according to ISO 8637-2 or
- in the form of a connecting piece (108B) for internal connection of a dialysis system as a port for dialysis liquid according to ISO 8637-1
and designed for coupling to a correspondingly designed counter-connecting piece (100), and
b. the connecting piece (100) has a fluid channel (111) surrounded by a conduction section (110) for circumferentially tight coupling in a plug-in direction (2) to the counter-connecting piece (100) and for subsequent exchange of liquids and/or gases with the counter-connecting piece, and
c. the connecting piece (100) has a transmission device (200, 300) for inductive data transmission with the counter-connecting piece,
**characterized by** one of the following additional features:
d. the transmission device (200, 300) has at least one planar coil (202, 302) surrounding the fluid channel (111), or
e. the transmission device (200, 300) has a helical coil (202, 302) surrounding the fluid channel (111).

2. Connecting piece according to Claim 1, having one of the following further features:
a. the planar coil (202, 302) is helical, or
b. the helical coil (202, 302) is cylindrical or conical.

3. Connecting piece according to Claim 1 or 2, having the following further feature:
a. the connecting piece (100) has a circumferential collar (120), wherein the planar coil (202, 302) is provided on or in the circumferential collar (120),
preferably having the following additional feature:
b. the circumferential collar (120) is integral with the conduction section (110).

4. Connecting piece according to Claim 1 or 2, having the following further feature:
a. the connecting piece has a sleeve-shaped ring section (130), on the inner side of which, on the outer side of which or within which the helical coil (202, 302) is provided,
preferably having at least one of the following additional features:
b. the sleeve-shaped ring section (130) is integral with the conduction section (110), wherein preferably the ring section (130) directly forms the conduction section (110) at least sectionally and wherein preferably the ring section (130) is preferably designed as a section which is conical at least sectionally on the outer side, and/or
c. the sleeve-shaped ring section (130) forms a coupling section (140), wherein a coupling device (142) is provided on the inner side or on the outer side, especially an internal thread, an external thread, a bayonet slot or a bayonet cam for engaging in a bayonet slot.

5. Connecting piece according to any of the preceding claims, having at least one of the following further features:
a. the connecting piece (100) is made from plastic at least in part, especially the conduction section (110) and/or the circumferential collar (120) and/or the sleeve-shaped ring section (130), and/or
b. the connecting piece (100) has at least one metallic or polymeric conductor (250) for conducting electrical energy or for conducting data.

6. Connecting piece (100) according to any of the preceding claims, having the following further feature:
a. the connecting piece (100) has at least one output device (160), preferably in the form of an optical output device (160) such as an LED.

7. Connecting piece (100) according to any of the preceding claims, having the following further feature:
a. the connecting piece (100) has an integrated circuit (170) which is supplied with power by the transmission device (300) and/or is connected to transmission device (200) for data transmission,
preferably having at least one of the following additional features:
b. the integrated circuit (170) has a unique identification code, and/or
c. the integrated circuit (170) has at least one digital or analogue input for evaluation of a sensor signal of a sensor (180), and/or
d. the integrated circuit has at least one digital or analogue output for activation of an actuator.

8. Connecting piece (100) according to any of the preceding claims, having the following further feature:
a. the connecting piece (100) has a sensor (180),
preferably having at least one of the following additional features:
b. the sensor is designed for capturing a property of a fluid flowing through or for capturing the volumetric flow rate of a fluid flowing through, or
c. the sensor is designed for capturing a coupling state of the connecting piece (100) on a counter-connecting piece.

9. Set of at least two connecting pieces (100), having the following features:
a. the two connecting pieces (100) are designed according to any of Claims 1 to 8 and for mechanical coupling to one another, and
b. the two connecting pieces (100) are designed in such a way that, through mechanical coupling, their respective fluid channels (111) are brought into communicating and circumferentially-tight connection with one another,
and
c. the two connecting pieces (100) are designed in such a way that, through mechanical coupling, their respective transmission devices (200, 300) are brought into a position relative to one another that allows data transmission or transmission of electrical energy.

10. Medical fluid line (400) for transferring liquids and/or gases, having the following features:
a. the fluid line (400) has a conduction body (410) in the form of a rigid pipe body or a flexible tube body that surrounds a fluid channel, and
b. the fluid line (400) has a connecting piece (100) at a proximal end of the conduction body (410), and
c. the connecting piece (100) is designed according to any of Claims 1 to 8.

11. Medical fluid line according to Claim 10, having at least one of the following further features:
a. the fluid line (400) has a connecting piece according to any of Claims 1 to 8 at a distal end, and/or
b. an electrically conductive conductor (250) for conducting digital data and/or for conducting electrical energy is provided along the conduction body (410), and/or
c. the conduction body (410) has a wall (412), within which the conductor (250) is arranged.

12. Fluid-guiding medical appliance (100), having the following features:
a. the appliance is designed for receiving, conducting and/or delivering fluids, and
b. the appliance is designed for receiving, conducting and/or delivering data and/or electrical energy, and
c. the appliance has a connecting piece according to any of Claims 1 to 8 for combined receiving, conduction and/or delivery of data and/or electrical energy and of a fluid.

13. Fluid-guiding medical appliance according to Claim 12, having at least one of the following further features:
a. the appliance has at least one actuator for controlling the fluid and/or applying pressure thereto, and/or
b. the appliance has at least one sensor, especially a sensor for capturing a property of the fluid or for capturing the volumetric flow rate of the fluid,
and/or
c. the appliance has at least one integrated circuit which is supplied with power by the transmission device and/or is connected to the transmission device for data transmission.

14. Fluid-guiding medical appliance according to either of Claims 12 and 13, having the following additional feature:
a. the appliance is designed as a conveying unit (500), or
b. the appliance is designed as a central venous catheter (520) or as a peripheral venous catheter (540), or
c. the appliance is designed as a valve bank (580) or multi-way valve (560), or
d. the appliance is designed as a haemodialysis cartridge, a haemodiafilter cartridge, a haemofilter or a haemoconcentrator cartridge (600), or
e. the appliance is designed as an auxiliary port device (620) and has a fluid line provided with a connecting piece on both sides, between which an auxiliary port for ad hoc addition of a fluid is provided, or
f. the appliance is designed as a filter appliance (640), or
g. the appliance is designed as a ventilation tube (610).

15. Medical fluid conduction and transfer system having the following features:
a. the system (10, 12, 14, 16) comprises an access unit for the patient (20) for administering a fluid or a fluid mixture into a patient (20) and/or for withdrawing a fluid from a patient, and
b. the system comprises at least one liquid container for providing or accommodating the fluid, and
c. the access unit and/or the liquid container has a connecting piece according to any of Claims 1 to 8.

## Revendications

1. Raccord (100) pour le couplage fluidique d'appareils médicaux et/ou de tubulures médicales, ayant les particularités suivantes :
a) le raccord (100) est réalisé
- à la manière d'un connecteur (101) selon la famille de normes ISO 80369, en particulier d'un connecteur pour les applications intravas-culaires ou hypodermiques ISO 80369-7 ou d'un connecteur pour les applications entérales selon ISO 80369-3 ou de connecteurs pour les applications neuraxiales selon ISO 80369-6, ou
- à la manière d'un bouchon (102A) pour flacons de perfusion selon ISO 8536-2, à la manière d'une capsule (102B) pour flacons de perfusion selon ISO 8536-3 ou à la manière d'un perforateur (103) selon ISO 8536-4 à raccorder à un flacon de perfusion ou à une poche, ou
- à la manière d'un port implanté par voie sous-cutanée (103) selon ISO 10555-6 ou à la manière d'une canule sans découpe (104) pour le couplage à un port implanté (104) selon ISO 10555-6, ou
- à la manière d'un raccord conique (107A) pour appareils d'anesthésie et respiratoires selon ISO 5356-1 ou à la manière d'un connecteur (107B) selon ISO 5367, ou
- à la manière d'un raccord (105, 106) pour les systèmes de drainage chirurgicaux selon ISO 20697, ou
- à la manière d'un raccord (108A) d'un circuit extracorporel d'un système d'hémodialyse, d'un système d'hémodiafiltration ou d'un système d'hémofiltre en tant que port sanguin selon ISO 8637-1 ou à la manière d'un raccord (108A) à raccorder à des ports sanguins pour systèmes de dialyse selon ISO 8637-2, ou
- à la manière d'un raccord (108B) pour la connexion interne d'un système de dialyse en tant que port pour le liquide de dialyse selon ISO 8637-1
et est réalisé pour le couplage à un raccord complémentaire (100) configuré de manière correspondante, et
b) le raccord (100) présente un canal de fluide (111) entouré d'une partie de tubulure (110) pour le couplage hermétique en circonférence dans une direction d'enfichage (2) au raccord complémentaire (100) et pour l'échange consécutif de liquides et/ou de gaz avec le raccord complémentaire, et
c) le raccord (100) présente un dispositif de transmission (200, 300) pour la transmission de données par induction avec le raccord complémentaire,
**caractérisé par** l'une des particularités supplémentaires suivantes :
d) le dispositif de transmission (200, 300) présente au moins une bobine plane (202, 302) qui est réalisée de façon à entourer le canal de fluide (111), ou
e) le dispositif de transmission (200, 300) présente une bobine (202, 302) hélicoïdale entourant le canal de fluide (111).

2. Raccord selon la revendication 1, ayant l'une des particularités supplémentaires suivantes :
a) la bobine plane (202, 302) est réalisée en forme de spirale, ou
b) la bobine hélicoïdale (202, 302) est réalisée de manière cylindrique ou conique.

3. Raccord selon la revendication 1 ou 2, ayant la particularité supplémentaire suivante :
a) le raccord (100) présente une collerette périphérique (120), la bobine plane (202, 302) étant prévue sur ou dans la collerette périphérique (120),
de préférence ayant la particularité supplémentaire suivante :
b) la collerette périphérique (120) est réalisée d'un seul tenant avec la partie de tubulure (110).

4. Raccord selon la revendication 1 ou 2, ayant la particularité supplémentaire suivante :
a) le raccord présente une partie annulaire (130) en forme de manchon sur la face intérieure de laquelle, sur la face extérieure de laquelle ou à l'intérieur de laquelle la bobine hélicoïdale (202, 302) est prévue,
de préférence ayant au moins l'une des particularités supplémentaires suivantes :
b) la partie annulaire (130) en forme de manchon est réalisée d'un seul tenant avec la partie de tubulure (110), dans lequel de préférence la partie annulaire (130) constitue la partie de tubulure (110) indirectement au moins par endroits, et dans lequel de préférence la partie annulaire (130) est réalisée de préférence comme une partie conique par endroits au moins en externe, et/ou
c) la partie annulaire (130) en forme de manchon constitue une partie de couplage (140), dans lequel un dispositif de couplage (142) est prévu sur la face intérieure ou sur la face extérieure, en particulier un filetage intérieur, un filetage extérieur, une coulisse à baïonnette ou une came à baïonnette pour venir en prise avec une coulisse à baïonnette.

5. Raccord selon l'une quelconque des revendications précédentes, ayant au moins l'une des particularités supplémentaires suivantes :
a) le raccord (100) est fabriqué au moins partiellement en matière plastique, en particulier la partie de tubulure (110) et/ou la collerette périphérique (120) et/ou la partie de tubulure (130) en forme de manchon, et/ou
b) le raccord (100) présente au moins un conducteur (250) métallique ou polymère pour l'acheminement d'énergie électrique ou de données.

6. Raccord (100) selon l'une quelconque des revendications précédentes, ayant la particularité supplémentaire suivante :
a) le raccord (100) présente au moins un dispositif de sortie (160), de préférence sous la forme d'un dispositif de sortie optique (160) tel qu'une LED.

7. Raccord (100) selon l'une quelconque des revendications précédentes, ayant la particularité supplémentaire suivante :
a) le raccord (100) présente un circuit intégré (170) qui est alimenté en courant par le dispositif de transmission (300) et/ou est relié au dispositif de transmission (200) pour la transmission de données,
de préférence ayant au moins l'une des particularités supplémentaires suivantes :
b) le circuit intégré (170) présente un code d'identification univoque, et/ou
c) le circuit intégré (170) dispose d'au moins une entrée numérique ou analogique pour évaluer un signal de capteur d'un capteur (180), et/ou
d) le circuit intégré dispose d'au moins une sortie numérique ou analogique pour piloter un actionneur.

8. Raccord (100) selon l'une quelconque des revendications précédentes, ayant la particularité supplémentaire suivante :
a) le raccord (100) présente un capteur (180),
de préférence ayant au moins l'une des particularités supplémentaires suivantes :
b) le capteur est réalisé pour détecter une propriété d'un fluide en écoulement ou pour détecter un débit volumique d'un fluide en écoulement, ou
c) le capteur est réalisé pour détecter un état de couplage du raccord (100) avec un raccord complémentaire.

9. Ensemble d'au moins deux raccords (100), ayant les particularités suivantes :
a) les deux raccords (100) sont réalisés selon l'une quelconque des revendications 1 à 8 et sont destinés au couplage mécanique mutuel, et
b) les deux raccords (100) sont réalisés de telle sorte que le couplage mécanique met en communication et en liaison hermétique en circonférence leurs canaux de fluide (111) respectifs, et
c) les deux raccords (100) sont réalisés de telle sorte que le couplage mécanique amène leurs dispositifs de transmission (200, 300) respectifs dans une position relative l'un par rapport à l'autre qui permet une transmission de données ou une transmission d'énergie électrique.

10. Tubulure de fluide médical (400) destinée à transporter des fluides et/ou des gaz, ayant les particularités suivantes :
a) la tubulure de fluide (400) présente un corps de tubulure (410) sous la forme d'un corps de tube rigide ou d'un corps de tuyau flexible qui entoure le canal de fluide, et
b) la tubulure de fluide (400) présente un raccord (100) à une extrémité proximale du corps de tubulure (410), et
c) le raccord (100) est réalisé selon l'une quelconque des revendications 1 à 8.

11. Tubulure de fluide médical selon la revendication 10, ayant au moins l'une des particularités supplémentaires suivantes :
a) la tubulure de fluide (400) présente à une extrémité distale un raccord selon l'une quelconque des revendications 1 à 8, et/ou
b) le long du corps de tubulure (410), un conducteur (250) électriquement conducteur est prévu pour l'acheminement de données numériques et/ou pour l'acheminement d'énergie électrique, et/ou
c) le corps de tubulure (410) présente une paroi (412) à l'intérieur de laquelle est disposé le conducteur (250).

12. Appareil médical conducteur de fluide (100), ayant les particularités suivantes :
a) l'appareil est réalisé pour recevoir, faire passer et/ou distribuer des fluides, et
b) l'appareil est réalisé pour recevoir, faire passer et/ou distribuer des données et/ou de l'énergie électrique, et
c) l'appareil présente un raccord selon l'une quelconque des revendications 1 à 8 pour recevoir, faire passer et/ou distribuer des données et/ou de l'énergie électrique ainsi qu'un fluide.

13. Appareil médical conducteur de fluide selon la revendication 12, ayant au moins l'une des particularités supplémentaires suivantes :
a) l'appareil présente au moins un actionneur pour commander et/ou mettre sous pression le fluide, et/ou
b) l'appareil présente au moins un capteur, en particulier un capteur pour détecter une propriété du fluide ou pour détecter le débit volumique du fluide, et/ou
c) l'appareil présente au moins un circuit intégré qui est alimenté en courant par le dispositif de transmission et/ou est relié au dispositif de transmission pour la transmission de données.

14. Appareil médical conducteur de fluide selon l'une quelconque des revendications 12 ou 13, ayant la particularité supplémentaire suivante :
a) l'appareil est réalisé sous la forme d'une unité de refoulement (500), ou
b) l'appareil est réalisé sous la forme d'un cathéter veineux (520) ou d'un cathéter veineux périphérique (540), ou
c) l'appareil est réalisé sous la forme de robinets de distribution (580) ou de robinet à voies multiples (560), ou
d) l'appareil est réalisé sous la forme d'une cartouche d'hémodialyse, d'une cartouche filtrante d'hémodialyse, d'un hémofiltre ou d'une cartouche d'hé-moconcentration (600), ou
e) l'appareil est réalisé sous la forme d'un dispositif de port supplémentaire (620) et dispose d'une tubulure de fluide munie d'un raccord des deux côtés entre lesquels un port supplémentaire est prévu pour ajouter un fluide au cas par cas, ou
f) l'appareil est réalisé sous la forme d'un appareil filtrant (640), ou
g) l'appareil est réalisé sous la forme d'un tube d'assistance respiratoire (610).

15. Système de guidage et de transfert de fluide médical, ayant les particularités suivantes :
a) le système (10, 12, 14, 16) comprend une unité d'accès au patient (20) pour appliquer un fluide ou un mélange de fluide chez un patient (20) et/ou pour l'extraction d'un fluide d'un patient, et
b) le système comprend au moins un récipient de liquide pour fournir ou recevoir le fluide, et
c) l'unité d'accès et/ou le récipient de liquide présente(nt) un raccord selon l'une quelconque des revendications 1 à 8.
